# EUROPEAN PATENT SPECIFICATION

(11) **EP 1 863 937 B1**
(45) Date of publication and mention of the grant of the patent: **22.09.2010**
(21) Application number: 06744682.3
(22) Date of filing: 23.03.2006
(51) Int. Cl.: C12Q 1/68

(54) **HUMAN AUTISM SUSCEPTIBILITY GENE ENCODING A TRANSMEMBRANE PROTEIN AND USES THEREOF**
EIN TRANSMEMBRANPROTEIN KODIERENDES HUMANES AUTISMUS-SUSZEPTIBILITÄTSGEN UND DESSEN VERWENDUNG
GENE HUMAIN DE SUSCEPTIBILITE A L'AUTISME CODANT UNE PROTEINE TRANSMEMBRANE ET SES UTILISATIONS

(30) Priority: 24.03.2005 US 664697 P
(43) Date of publication of application: 12.12.2007
(73) Proprietor: Integragen, 91000 EVRY (FR)
(72) Inventor: PHILIPPI, Anne, F-77310 St. Fargeau Ponthierry (FR); ROUSSEAU, Francis, F-91600 Savigny sur Orge (FR); ROSCHMANN, Elke, 89179 Beimerstetten (DE)
(74) Representative: Chajmowicz, Marion
(86) International application number: PCT/IB2006/001219
(87) International publication number: WO 2006/100608

(56) References cited:
- WO-A-00/53802
- WO-A-2004/033717
- US-A1- 2003 096 264
- SPENCE SARAH J: "The genetics of autism." SEMINARS IN PEDIATRIC NEUROLOGY. SEP 2004, vol. 11, no. 3, September 2004 (2004-09), pages 196-204, XP004666241 ISSN: 1071-9091
- MUHLE REBECCA ET AL: "The genetics of autism." PEDIATRICS. MAY 2004, vol. 113, no. 5, May 2004 (2004-05), pages e472-e486, XP002388697 ISSN: 1098-4275
- SHASTRY B S: "Molecular genetics of autism spectrum disorders" JOURNAL OF HUMAN GENETICS, vol. 48, no. 10, 2003, pages 495-501, XP002315920
- LAURITSEN M B ET AL: "The genetics of autism" ACTA PSYCHIATRICA SCANDINAVICA, MUNKSGAARD, COPENHAGEN, DE, vol. 103, no. 6, June 2001 (2001-06), pages 411-427, XP002358261 ISSN: 0001-690X
- HUSSMAN JOHN P: "Suppressed GABAergic inhibition as a common factor in suspected etiologies of autism" JOURNAL OF AUTISM AND DEVELOPMENTAL DISORDERS, vol. 31, no. 2, April 2001 (2001-04), pages 247-248, XP002388698 ISSN: 0162-3257 cited in the application
- BROOKS P ET AL: "GENOME WIDE MAPPING OF AUTISM SUSCEPTIBILITY LOCI BY ENZYMATIC SELECTION OF IDENTITY-BY-DESCENT REGIONS IN AUTISTIC SIBLING PAIRS" AMERICAN JOURNAL OF MEDICAL GENETICS, WILEY, NEW YORK,NY, US, vol. 130B, no. 1, September 2004 (2004-09), page 29, XP009058507 ISSN: 0148-7299
- LANDER E.; KRUGLYAK L.: 'Genetic dissection of complex traits: guidelines for interpreting and reporting linkage results' NATURE GENETICS vol. 11, November 1995, pages 241 - 247

## Description

### FIELD OF THE INVENTION

The present invention relates generally to the fields of genetics and medicine.

### BACKGROUND OF THE INVENTION

Autism is a neuropsychiatric developmental disorder characterized by impairments in reciprocal social interaction and verbal and non-verbal communication, restricted and stereotyped patterns of interests and activities, and the presence of developmental abnormalities by 3 years of age (Bailey et al., 1996). In his pioneer description of infantile autism, Kanner (1943) included the following symptoms: impaired language, lack of eye contact, lack of social interaction, repetitive behavior, and a rigid need for routine. He noted that in most cases the child's behavior was abnormal from early infancy. On this basis, he suggested the presence of an inborn, presumably genetic, defect. One year later, Hans Asperger in Germany described similar patients and termed the condition "autistic psychopathy".

Autism is defined using behavioral criteria because, so far, no specific biological markers are known for diagnosing the disease. The clinical picture of autism varies in severity and is modified by many factors, including education, ability and temperament. Furthermore, the clinical picture changes over the course of the development within an individual. In addition, autism is frequently associated with other disorders such as attention deficit disorder, motor in coordination and psychiatric symptoms such as anxiety and depression. There is some evidence that autism may also encompass epileptic, metabolic and immune disorder. In line with the clinical recognition of the variability, there is now general agreement that there is a spectrum of autistic disorders, which includes individuals at all levels of intelligence and language ability and spanning all degrees of severity.

Part of the autism spectrum, but considered a special subgroup, is Asperger syndrome (AS). AS is distinguished from autistic disorder by the lack of a clinically significant delay in language development in the presence of the impaired social interaction and restricted repetitive behaviors, interests, and activities that characterize the autism spectrum disorders (ASDs).

ASDs are types of pervasive developmental disorders (PPD). PPD, "not otherwise specified" (PPD-NOS) is used to categorize children who do not meet the strict criteria for autism but who come close, either by manifesting atypical autism or by nearly meeting the diagnostic criteria in two or three of the key areas.

To standardize the diagnosis of autism, diagnostic criteria have been defined by the World Health Organisation (International Classification of Diseases, 10^{th} Revision (ICD-10), 1992) and the American Psychiatric Association (Diagnostic and Statistical Manual of Mental Disorders, 4^{th} edition (DSM-IV), 1994). An Autism Diagnostic Interview (ADI) has been developed (Le Couteur et al., 1989; Lord et al., 1994). The ADI is the only diagnostic tool available to diagnose ASD that has been standardized, rigorously tested and is universally recognized. The ADI is a scored, semi-structured interview of parents that is based on ICD-10 and DSM-IV criteria for the diagnosis of autism. It focuses on behavior in three main areas: qualities of reciprocal social interaction; communication and language; and restricted and repetitive, stereotyped interests and behaviors. Using these criteria, autism is no longer considered a rare disorder. Higher rates of 10-12 cases per 10,000 individuals have been reported in more recent studies (Gillberg and Wing, 1999) compared to the previously reported prevalence rate of 4-5 patients per 10,000 individuals based on Kanner's criteria (Folstein and Rosen-Sheidley, 2001). Estimates for the prevalence rate of the full spectrum of autistic disorders are 1.5 to 2.5 times higher. Reports of a four times higher occurrence in males compared to females are consistent. Mental retardation is present in between 25% and 40% of cases with ASD (Baird et al. 2000; Chakrabarti and Fombonne, 2001). Additional medical conditions involving the brain are seen in ca. 10% of the population (Gillberg and Coleman, 2000).

The mechanisms underlying the increase in reported cases of autism are unknown. It is highly debated whether this difference reflects an increase in the prevalence of autism, a gradual change in diagnostic criteria, a recognition of greater variability of disease expression, or an increased awareness of the disorder. In addition, there is a widespread public perception that the apparent increase is due primarily to environmentally factors (Nelson, 1991; Rodier and Hyman, 1998). However, it seems likely that most of the increased prevalence can be explained by a broadening of the diagnostic criteria, in combination with a broader application of these criteria.

Although there are effective treatments for ameliorating the disease, there are no cures available and benefits of treatment tend to be modest. Promising results have been obtained for several programs utilizing various behavioral and developmental strategies. Among the most promising are programs based on applied behavior analysis (ABA). Several medications appeared to improve various symptoms associated with autism, thereby increasing individuals' ability to benefit from educational and behavioral interventions. The most extensively studied agents are the dopamine antagonists. Several studies suggest the usefulness of various selective serotonin reuptake inhibitors.

A review entitled "The genetics of autism" has been published by S.J Spence (Seminars in Pediatric Neurology, vol. 11, no. 3, September 2004 (2004-09) , pages 196-204).

Three twin studies have been performed to estimate heritability of autism (Folstein and Rutter, 1977; Bailey et al., 1995; Steffenburg et al., 1989). All twins who lived in a geographically defmed population were sought out. In the combined data 36 monozygotic (MZ) and 30 dizygotic (DZ) twins were studied. The average MZ concordance rate is 70% compared to a DZ rate of 0%. A heritability of more than 90% was calculated from the MZ to DZ concordance ratio and the sibling recurrence risk that has been estimated to be ca 2%-4% (Jorde et al., 1991 Szatmari et al., 1998). Studies of non-autistic relatives have clearly shown that several characteristics of the ASDs are found more often in the parents of autistic children than the parents of controls including social reticence, communication difficulties, preference for routines and difficulty with change (Folstein and Rutter, 1977). Delayed onset of speech and difficulty with reading are also more common in family members of individuals with autism, as are recurrent depression, anxiety disorders, elevated platelet serotonin and increased head circumference (Folstein and Rosen-Sheidley, 2001).

The incidence of autism falls significantly with decreasing degree of relatedness to an affected individual indicating that a single-gene model is unlikely to account for most cases of autism (Jorde et al., 1990). A reported segregation analysis was most consistent with a polygenic mode of inheritance (Jorde et al., 1991). The most parsimonious genetic model is one in which several genes interact with one another to produce the autism phenotype (Folstein and Rosen-Sheidley, 2001).

Considerable indirect evidence indicates a possible role for autoimmunity in autism. One study found more family members with autoimmune diseases in families with an autistic proband compared with control probands (Comi et al., 1999). A few studies reported that haplotypes at the Major Histocompatibility Complex (MHC) locus present in some children with autism, or their mothers, might predipose their autistic children to autoimmunity (Burger and Warren, 1998). In two studies, autoantibodies to certain brain tissues and proteins, including myelin basic protein, neurofilament proteins and vascular epithelium were found more often in autistic children compared to controls (Singh et al., 1993; Connolly et al., 1999; Weizman et al., 1982).

Although most autism cases are consistent with the proposed mechanism of oligogenicity and epistasis, a minority have been seen in association with chromosomal abnormalities and with disorders that have specific etiologies. Smalley (1997) stated that approximately 15 to 37% of cases of autism have a comorbid medical condition, including 5 to 14% with a known genetic disorder or chromosomal anomaly. Chromosome anomalies involving almost all human chromosomes have been reported. These include autosomal aneuploidies, sex-chromosome anomalies, deletions, duplications, translocations, ring chromosomes, inversions and marker chromosomes (Gillberg, 1998). Most common are abnormalities of the Prader Willi/Angelman Syndrome region on chromosome 15. Association of autism and a Mendelian condition or genetic syndrome included untreated phenylketonuria, fragile X syndrome, tuberous sclerosis and neurofibromatosis. Recently, Carney et al. (2003) identified mutations in the MECP2 (methyl CpG-binding protein 2) gene in two females with autism who do not have manifestations of Rett syndrome caused in 80% of the cases by mutations in the MECP2 gene.

Different groups are conducting genome scans related to autism or the broader phenotypes of ASDs. This approach appears very promising, because it is both systematic and model free. In addition, it has already been shown to be successful. Thus, positive linkage results have been obtained even by analysing comparatively small study groups. More important, some findings have already been replicated. The most consistent result was obtained for chromosome 7q, but there is also considerable overlap on chromosomes 2q and 16p (Folstein and Rosen-Sheidley, 2001). Considerable progress in identifying chromosomal regions have also been made on chromosome 15 and X. Mutations in two X-linked genes encoding neuroligins NLGN3 and NLGN4 have been identified in siblings with autism spectrum disorders (Jamain et al., 2003). Several lines of evidence support the fact that mutations in neuroligins are involved in autistic disorder. First, the reported mutations cause severe alterations of the predicted protein structure. Second, deletions at Xp22.3 that include NLGN4 have been reported in several autistic children. Third, a mutation in NLGN4 appeared *de novo* in one affected individual's mother.

### SUMMARY OF THE INVENTION

The present invention now discloses the identification of a human autism susceptibility gene, which can be used for the diagnosis of autism, as well as for the screening of therapeutically active drugs.

The present invention more particularly discloses the identification of a human autism susceptibility gene, which can be used for the diagnosis of autism, as well as for the screening of therapeutically active drugs. The invention more specifically discloses certain alleles of the ATPase, Ca++ transporting, plasma membrane 2 (ATP2B2) gene related to susceptibility to autism and representing novel targets for therapeutic intervention. The present invention relates to particular mutations in the ATP2B2 gene and expression products, as well as to diagnostic tools and kits based on these mutations.

The invention can be used in the diagnosis of predisposition to or protection from, detection of autism, the method comprising detecting in a sample from the subject the presence of an alteration in the ATP2B2 gene, the presence of said alteration being indicative of the presence or predisposition to autism. The presence of said alteration can also be indicative for protecting from autism.

A particular object of this invention resides in a method of detecting the presence of or predisposition to autism in a subject, the method comprising detecting the presence of an alteration in the ATP2B2 gene in a sample from the subject, the presence of said alteration being indicative of the presence of or the predisposition to autism.

An additional particular object of this invention resides in a method of detecting the protection from autism in a subject, the method comprising detecting the presence of an alteration in the ATP2B2 gene in a sample from the subject, the presence of said alteration being indicative of the protection from autism.

Another particular object described resides in a method of assessing the response of a subject to a treatment of autism, the method comprising detecting the presence of an alteration in the ATP2B2 gene in a sample from the subject, the presence of said alteration being indicative of a particular response to said treatment.

A further particular object described resides in a method of assessing the adverse effect in a subject to a treatment of autism, the method comprising detecting the presence of an alteration in the ATP2B2 gene in a sample from the subject, the presence of said alteration being indicative of an adverse effect to said treatment.

In a preferred embodiment, said alteration is one or several SNP(s) or a haplotype of SNPs associated with autism. More preferably, said haplotype associated with autism comprises or consists of several SNPs selected from the group consisting of SNP21, SNP22, SNP28, SNP39, SNP46, SNP61, SNP73 and SNP74. Still more preferably, said haplotype is selected from the haplotypes disclosed in Table 4. More preferably, said SNP associated with autism can be SNP22.

Preferably, the alteration in the ATP2B2 gene locus is determined by performing a hydridization assay, a sequencing assay, a microsequencing assay, or an allele-specific amplification assay.

A particular aspect of this disclosure resides in compositions of matter comprising primers, probes, and/or oligonucleotides, which are designed to specifically detect at least one SNP or haplotype associated with autism in the genomic region including the ATP2B2 gene, or a combination thereof. More preferably, said haplotype associated with autism comprises or consists of several SNPs selected from the group consisting of SNP21, SNP22, SNP28, SNP39, SNP46, SNP61, SNP73 and SNP74. Still more preferably, said haplotype is selected from the haplotypes disclosed in Table 4. More preferably, said SNP associated with autism can be SNP22.

A further aspect of this invention resides in the screening of drugs for therapy of autism, based on the modulation of or binding to an allele of ATP2B2 gene associated with autism or gene product thereof.

### LEGEND TO THE FIGURES

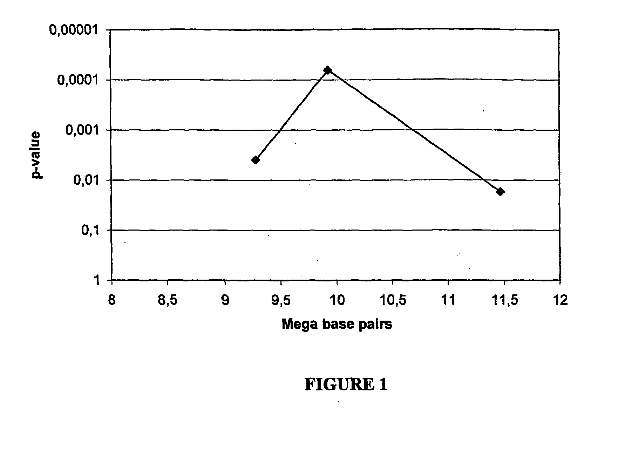

Figure 1 : High density mapping using Genomic Hybrid Identity Profiling (GenomeHIP).

### DETAILED DESCRIPTION OF THE INVENTION

The present invention discloses the identification of ATP2B2 as a human autism susceptibility gene. Various nucleic acid samples from 114 families with autism were submitted to a particular GenomeHIP process. This process led to the identification of particular identical-by-descent fragments in said populations that are altered in autistic subjects. By screening of the IBD fragments, we identified the ATPase, Ca++ transporting, plasma membrane 2 gene on chromosome 3p25.3 (ATP2B2) as a candidate for autism and related phenotypes. This gene is indeed present in the critical interval and expresses a functional phenotype consistent with a genetic regulation of autism. SNPs of the ATP2B2 gene were also identified, as being correlated to autism in human subjects. SNP22, located in the ATP2B2 gene locus was found to be associated with autism. Haplotypes disclosed in Table 4 comprising several SNPs selected from the group consisting of SNP21, SNP22, SNP28, SNP39, SNP46, SNP61, SNP73 and SNP74 have also been identified as associated with autism.

The present invention thus proposes to use ATP2B2 gene and corresponding expression products for the diagnosis, prevention and treatment of autism, as well as for the screening of therapeutically active drugs.

### DEFINITIONS

Autism and autism spectrum disorders (ASDs): Autism is typically characterized as part of a spectrum of disorders (ASDs) including Asperger syndrome (AS) and other pervasive developmental disorders (PPD). Autism shall be construed as any condition of impaired social interaction and communication with restricted repetitive and stereotyped patterns of behavior, interests and activities present before the age of 3, to the extent that health may be impaired. AS is distinguished from autistic disorder by the lack of a clinically significant delay in language development in the presence of the impaired social interaction and restricted repetitive behaviors, interests, and activities that characterize the autism-spectrum disorders (ASDs). PPD-NOS (PPD, not otherwise specified) is used to categorize children who do not meet the strict criteria for autism but who come close, either by manifesting atypical autism or by nearly meeting the diagnostic criteria in two or three of the key areas.

The invention may be used in various subjects, particularly human, including adults, children and at the prenatal stage.

Within the context of this invention, the ATP2B2 gene locus designates all ATP2B2 sequences or products in a cell or organism, including ATP2B2 coding sequences, ATP2B2 non-coding sequences (e.g., introns), ATP2B2 regulatory sequences controlling transcription, translation (e.g., promoter, enhancer, terminator, etc.), RNA and/or protein stability, as well as all corresponding expression products, such as ATP2B2 RNAs (e.g., mRNAs) and ATP2B2 polypeptides (e.g., a pre-protein and a mature protein). The ATP2B2 gene locus also comprise surrounding sequences of the ATP2B2 gene which include SNPs that are in linkage disequilibrium with SNPs located in the ATP2B2 gene.

As used in the present application, the term "ATP2B2 gene" designates the ATPase, Ca++ transporting, plasma membrane 2 gene on human chromosome 3p25.3, as well as variants, analogs and fragments thereof, including alleles thereof (e.g., germline mutations) which are related to susceptibility to autism. The ATP2B2 gene may also be referred to as PMCA2.

The term "gene" shall be construed to include any type of coding nucleic acid, including genomic DNA (gDNA), complementary DNA (cDNA), synthetic or semi-synthetic DNA, as well as any form of corresponding RNA. The term gene particularly includes recombinant nucleic acids encoding ATP2B2, i.e., any non naturally occurring nucleic acid molecule created artificially, e.g., by assembling, cutting, ligating or amplifying sequences. An ATP2B2 gene is typically double-stranded, although other forms may be contemplated, such as single-stranded. ATP2B2 genes may be obtained from various sources and according to various techniques known in the art, such as by screening DNA libraries or by amplification from various natural sources. Recombinant nucleic acids may be prepared by conventional techniques, including chemical synthesis, genetic engineering, enzymatic techniques, or a combination thereof. Suitable ATP2B2 gene sequences may be found on gene banks, such as Unigene Cluster for ATP2B2 (Hs. 268942) and Unigene Representative Sequence NM_001001331. A particular example of an ATP2B2 gene comprises SEQ ID No: 1.

The term "ATP2B2 gene" includes any variant, fragment or analog of SEQ ID No 1 or of any coding sequence as identified above. Such variants include, for instance, naturally-occurring variants due to allelic variations between individuals (e.g., polymorphisms), mutated alleles related to autism, alternative splicing forms, etc. The term variant also includes ATP2B2 gene sequences from other sources or organisms. Variants are preferably substantially homologous to SEQ ID No 1, i.e., exhibit a nucleotide sequence identity of at least about 65%, typically at least about 75%, preferably at least about 85%, more preferably at least about 95% with SEQ ID No 1. Variants and analogs of an ATP2B2 gene also include nucleic acid sequences, which hybridize to a sequence as defined above (or a complementary strand thereof) under stringent hybridization conditions.

Typical stringent hybridisation conditions include temperatures above 30° C, preferably above 35°C, more preferably in excess of 42°C, and/or salinity of less than about 500 mM, preferably less than 200 mM. Hybridization conditions may be adjusted by the skilled person by modifying the temperature, salinity and/or the concentration of other reagents such as SDS, SSC, etc.

A fragment of an ATP2B2 gene designates any portion of at least about 8 consecutive nucleotides of a sequence as disclosed above, preferably at least about 15, more preferably at least about 20 nucleotides, further preferably of at least 30 nucleotides. Fragments include all possible nucleotide lengths between 8 and 100 nucleotides, preferably between 15 and 100, more preferably between 20 and 100.

An ATP2B2 polypeptide designates any protein or polypeptide encoded by an ATP2B2 gene as disclosed above. The term "polypeptide" refers to any molecule comprising a stretch of amino acids. This term includes molecules of various lengths, such as peptides and proteins. The polypeptide may be modified, such as by glycosylations and/or acetylations and/or chemical reaction or coupling, and may contain one or several non-natural or synthetic amino acids. A specific example of an ATP2B2 polypeptide comprises all or part of SEQ ID No: 2 (NP_001001331).

The terms "response to a treatment" refer to treatment efficacy, including but not limited to ability to metabolise a therapeutic compound, to the ability to convert a pro-drug to an active drug, and to the pharmacokinetics (absorption, distribution, elimination) and the pharmacodynamics (receptor-related) of a drug in an individual.

The terms "adverse effects to a treatment" refer to adverse effects of therapy resulting from extensions of the principal pharmacological action of the drug or to idiosyncratic adverse reactions resulting from an interaction of the drug with unique host factors. "Side effects to a treatment" include, but are not limited to, adverse reactions such as dermatologic, hematologic or hepatologic toxicities and further includes gastric and intestinal ulceration, disturbance in platelet function, renal injury, generalized urticaria, bronchoconstriction, hypotension, and shock.

### DIAGNOSIS

The invention now provides diagnosis methods based on a monitoring of the ATP2B2 gene locus in a subject. Within the context of the present invention, the term 'diagnosis" includes the detection, monitoring, dosing, comparison, etc., at various stages, including early, pre-symptomatic stages, and late stages, in adults, children and pre-birth. Diagnosis typically includes the prognosis, the assessment of a predisposition or risk of development, the characterization of a subject to define most appropriate treatment (pharmacogenetics), etc. The present invention provides diagnostic methods to determine whether an individual is at risk of developing autism, or suffers from autism, resulting from a mutation or a polymorphism in the ATP2B2 gene. The present invention also provides methods to determine whether an individual is likely to respond positively to a therapeutic agent or whether an individual is at risk of developing an adverse side effect to a therapeutic agent.

A particular object of this invention resides in a method of detecting the presence of or predisposition to autism, the method comprising detecting in a sample from the subject the presence of an alteration in the ATP2B2 gene in said sample. The presence of said alteration is indicative of the presence or predisposition to autism. Optionally, said method comprises a previous step of providing a sample from a subject. Preferably, the presence of an alteration in the ATP2B2 gene in said sample is detected through the genotyping of a sample.

Another particular object of this invention resides in a method of detecting the protection from autism in a subject, the method comprising detecting the presence of an alteration in the ATP2B2 gene in a sample from the subject, the presence of said alteration being indicative of the protection from autism.

In a preferred embodiment, said alteration is one or several SNP(s) or a haplotype of SNPs associated with autism. More preferably, said haplotype associated with autism comprises or consists of several SNPs selected from the group consisting of SNP21, SNP22, SNP28, SNP39, SNP46, SNP61, SNP73 and SNP74. Still more preferably, said haplotype is selected from the haplotypes disclosed in Table 4. More preferably, said SNP associated with autism is SNP22.

Another particular object described resides in a method of assessing the response of a subject to a treatment of autism , the method comprising (i) providing a sample from the subject and (ii) detecting the presence of an alteration in the ATP2B2 gene in said sample.

Another particular object described resides in a method of assessing the response of a subject to a treatment of autism , the method comprising detecting in a sample from the subject the presence of an alteration in the ATP2B2 gene in said sample. The presence of said alteration is indicative of a particular response to said treatment. Preferably, the presence of an alteration in the ATP2B2 gene in said sample is detected through the genotyping of a sample.

A further particular object described resides in a method of assessing the adverse effects of a subject to a treatment of autism , the method comprising detecting in a sample from the subject the presence of an alteration in the ATP2B2 gene in said sample. The presence of said alteration is indicative of adverse effects to said treatment. Preferably, the presence of an alteration in the ATP2B2 gene in said sample is detected through the genotyping of a sample.

In a preferred embodiment, said alteration is one or several SNP(s) or a haplotype of SNPs associated with autism. More preferably, said haplotype associated with autism comprises or consists of several SNPs selected from the group consisting of SNP21, SNP22, SNP28, SNP39, SNP46, SNP61, SNP73 and SNP74. Still more preferably, said haplotype is selected from the haplotypes disclosed in Table 4. More preferably, said SNP associated with autism is SNP22.

Diagnostics, which analyse and predict response to a treatment or drug, or side effects to a treatment or drug, may be used to determine whether an individual should be treated with a particular treatment drug. For example, if the diagnostic indicates a likelihood that an individual will respond positively to treatment with a particular drug, the drug may be administered to the individual. Conversely, if the diagnostic indicates that an individual is likely to respond negatively to treatment with a particular drug, an alternative course of treatment may be prescribed. A negative response may be defined as either the absence of an efficacious response or the presence of toxic side effects.

Clinical drug trials represent another application for the ATP2B2 SNPs. One or more ATP2B2 SNPs indicative of response to a drug or to side effects to a drug may be identified using the methods described above. Thereafter, potential participants in clinical trials of such an agent may be screened to identify those individuals most likely to respond favorably to the drug and exclude those likely to experience side effects. In that way, the effectiveness of drug treatment may be measured in individuals who respond positively to the drug, without lowering the measurement as a result of the inclusion of individuals who are unlikely to respond positively in the study and without risking undesirable safety problems.

The alteration may be determined at the level of the ATP2B2 gDNA, RNA or polypeptide. Optionally, the detection is performed by sequencing all or part of the ATP2B2 gene or by selective hybridisation or amplification of all or part of the ATP2B2 gene. More preferably an ATP2B2 gene specific amplification is carried out before the alteration identification step.

An alteration in the ATP2B2 gene locus may be any form of mutation(s), deletion(s), rearrangement(s) and/or insertions in the coding and/or non-coding region of the locus, alone or in various combination(s). Mutations more specifically include point mutations. Deletions may encompass any region of two or more residues in a coding or non-coding portion of the gene locus, such as from two residues up to the entire gene or locus. Typical deletions affect smaller regions, such as domains (introns) or repeated sequences or fragments of less than about 50 consecutive base pairs, although larger deletions may occur as well. Insertions may encompass the addition of one or several residues in a coding or non-coding portion of the gene locus. Insertions may typically comprise an addition of between 1 and 50 base pairs in the gene locus. Rearrangement includes inversion of sequences. The ATP2B2 gene locus alteration may result in the creation of stop codons, frameshift mutations, amino acid substitutions, particular RNA splicing or processing, product instability, truncated polypeptide production, etc. The alteration may result in the production of an ATP2B2 polypeptide with altered function, stability, targeting or structure. The alteration may also cause a reduction in protein expression or, alternatively, an increase in said production.

In a particular embodiment of the method according to the present invention, the alteration in the ATP2B2 gene locus is selected from a point mutation, a deletion and an insertion in the ATP2B2 gene or corresponding expression product, more preferably a point mutation and a deletion. The alteration may be determined at the level of the ATP2B2 gDNA, RNA or polypeptide.

In this regard, the present invention now discloses a SNP in the ATP2B2 gene and certain haplotypes, which include SNPs selected from the group consisting of SNP21, SNP22, SNP28, SNP39, SNP46, SNP61, SNP73 and SNP74, that are associated with autism. The SNPs are reported in the following Table 1.

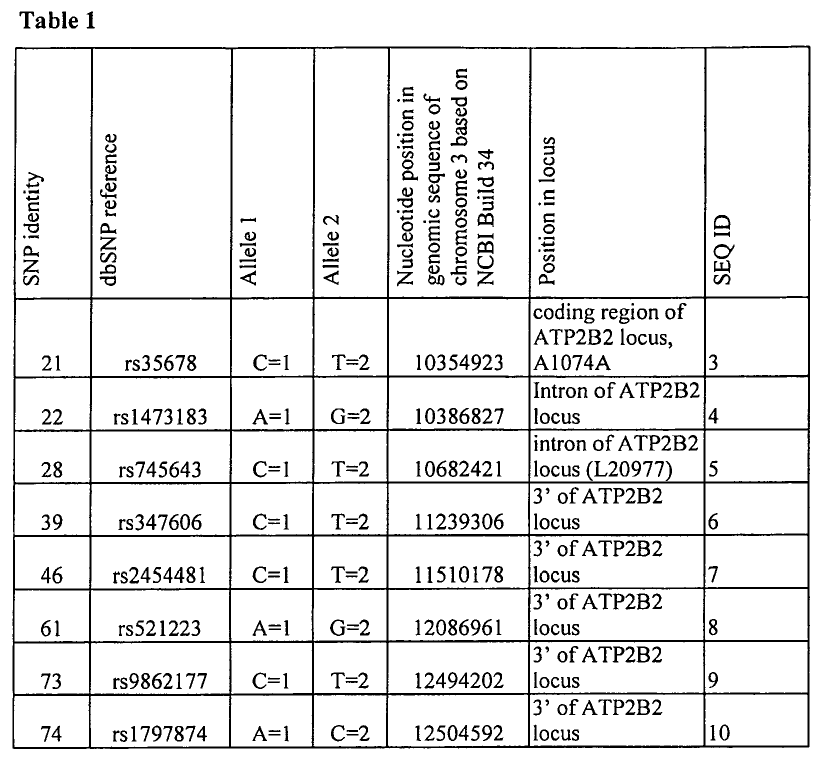

In any method according to the present invention, one or several SNPs in the ATP2B2 gene and certain haplotypes comprising SNPs in the ATP2B2 gene and surrounding regions, more particularly SNP21, SNP22, SNP28, SNP39, SNP46, SNP61, SNP73 and SNP74, can be used in combination with another SNP or haplotype associated with autism and located in other gene(s).

In another variant, the method comprises detecting the presence of an altered ATP2B2 RNA expression. Altered RNA expression includes the presence of an altered RNA sequence, the presence of an altered RNA splicing or processing, the presence of an altered quantity of RNA, etc. These may be detected by various techniques known in the art, including by sequencing all or part of the ATP2B2 RNA or by selective hybridisation or selective amplification of all or part of said RNA, for instance.

In a further variant, the method comprises detecting the presence of an altered ATP2B2 polypeptide expression. Altered ATP2B2 polypeptide expression includes the presence of an altered polypeptide sequence, the presence of an altered quantity of ATP2B2 polypeptide, the presence of an altered tissue distribution, etc. These may be detected by various techniques known in the art, including by sequencing and/or binding to specific ligands (such as antibodies), for instance.

As indicated above, various techniques known in the art may be used to detect or quantify altered ATP2B2 gene or RNA expression or sequence, including sequencing, hybridisation, amplification and/or binding to specific ligands (such as antibodies). Other suitable methods include allele-specific oligonucleotide (ASO), allele-specific amplification, Southern blot (for DNAs), Northern blot (for RNAs), single-stranded conformation analysis (SSCA), PFGE, fluorescent in situ hybridization (FISH), gel migration, clamped denaturing gel electrophoresis, heteroduplex analysis, RNase protection, chemical mismatch cleavage, ELISA, radio-immunoassays (RIA) and immuno-enzymatic assays (IEMA).

Some of these approaches (e.g., SSCA and CGGE) are based on a change in electrophoretic mobility of the nucleic acids, as a result of the presence of an altered sequence. According to these techniques, the altered sequence is visualized by a shift in mobility on gels. The fragments may then be sequenced to confirm the alteration.

Some others are based on specific hybridisation between nucleic acids from the subject and a probe specific for wild type or altered ATP2B2 gene or RNA. The probe may be in suspension or immobilized on a substrate. The probe is typically labeled to facilitate detection of hybrids.

Some of these approaches are particularly suited for assessing a polypeptide sequence or expression level, such as Northern blot, ELISA and RIA. These latter require the use of a ligand specific for the polypeptide, more preferably of a specific antibody.

In a particular, preferred, embodiment, the method comprises detecting the presence of an altered ATP2B2 gene expression profile in a sample from the subject. As indicated above, this can be accomplished more preferably by sequencing, selective hybridisation and/or selective amplification of nucleic acids present in said sample.

### Sequencing

Sequencing can be carried out using techniques well known in the art, using automatic sequencers. The sequencing may be performed on the complete ATP2B2 gene or, more preferably, on specific domains thereof, typically those known or suspected to carry deleterious mutations or other alterations.

### Amplification

Amplification is based on the formation of specific hybrids between complementary nucleic acid sequences that serve to initiate nucleic acid reproduction.

Amplification may be performed according to various techniques known in the art, such as by polymerase chain reaction (PCR), ligase chain reaction (LCR), strand displacement amplification (SDA) and nucleic acid sequence based amplification (NASBA). These techniques can be performed using commercially available reagents and protocols. Preferred techniques use allele-specific PCR or PCR-SSCP. Amplification usually requires the use of specific nucleic acid primers, to initiate the reaction.

Nucleic acid primers useful for amplifying sequences from the ATP2B2 gene or locus are able to specifically hybridize with a portion of the ATP2B2 gene locus that flank a target region of said locus, said target region being altered in certain subjects having autism. Examples of such target regions are provided in Table 1.

Primers that can be used to amplify ATP2B2 target region comprising SNPs as identified in Table 1 may be designed based on the sequence of Seq Id No 1 or on the genomic sequence of ATP2B2. In a particular embodiment, primers may be designed based on the sequence of SEQ ID Nos 3-10.

Another particular object of this disclosure resides in a nucleic acid primer useful for amplifying sequences from the ATP2B2 gene or locus including surrounding regions. Such primers are preferably complementary to, and hybridize specifically to nucleic acid sequences in the ATP2B2 gene locus. Particular primers are able to specifically hybridise with a portion of the ATP2B2 gene locus that flank a target region of said locus, said target region being altered in certain subjects having autism.

The disclosure also relates to a nucleic acid primer, said primer being complementary to and hybridizing specifically to a portion of an ATP2B2 coding sequence (e.g., gene or RNA) altered in certain subjects having autism. In this regard, particular primers of this invention are specific for altered sequences in an ATP2B2 gene or RNA. By using such primers, the detection of an amplification product indicates the presence of an alteration in the ATP2B2 gene locus. In contrast, the absence of amplification product indicates that the specific alteration is not present in the sample.

Typical primers of this disclosure are single-stranded nucleic acid molecules of about 5 to 60 nucleotides in length, more preferably of about 8 to about 25 nucleotides in length. The sequence can be derived directly from the sequence of the ATP2B2 gene locus. Perfect complementarity is preferred, to ensure high specificity. However, certain mismatch may be tolerated.

The disclosure also concerns the use of a nucleic acid primer or a pair of nucleic acid primers as described above in a method of detecting the presence of or predisposition to autism, in a subject or in a method of assessing the response of a subject to a treatment of autism.

### Selective hybridization

Hybridization detection methods are based on the formation of specific hybrids between complementary nucleic acid sequences that serve to detect nucleic acid sequence alteration(s).

A particular detection technique involves the use of a nucleic acid probe specific for wild type or altered ATP2B2 gene or RNA, followed by the detection of the presence of a hybrid. The probe may be in suspension or immobilized on a substrate or support (as in nucleic acid array or chips technologies). The probe is typically labeled to facilitate detection of hybrids.

In this regard, a particular embodiment of this invention comprises contacting the sample from the subject with a nucleic acid probe specific for an altered ATP2B2 gene locus, and assessing the formation of an hybrid. In a particular, preferred embodiment, the method comprises contacting simultaneously the sample with a set of probes that are specific, respectively, for wild type ATP2B2 gene locus and for various altered forms thereof. In this embodiment, it is possible to detect directly the presence of various forms of alterations in the ATP2B2 gene locus in the sample. Also, various samples from various subjects may be treated in parallel.

Within the context of this disclosure, a probe refers to a polynucleotide sequence which is complementary to and capable of specific hybridisation with a (target portion of a) ATP2B2 gene or RNA, and which is suitable for detecting polynucleotide polymorphisms associated with ATP2B2 alleles which predispose to or are associated with autism. Probes are preferably perfectly complementary to the ATP2B2 gene, RNA, or target portion thereof. Probes typically comprise single-stranded nucleic acids of between 8 to 1000 nucleotides in length, for instance of between 10 and 800, more preferably of between 15 and 700, typically of between 20 and 500. It should be understood that longer probes may be used as well. A preferred probe of this invention is a single stranded nucleic acid molecule of between 8 to 500 nucleotides in length, which can specifically hybridise to a region of an ATP2B2 gene or RNA that carries an alteration.

A specific embodiment of this disclosure is a nucleic acid probe specific for an altered (e.g., a mutated) ATP2B2 gene or RNA, i.e., a nucleic acid probe that specifically hybridises to said altered ATP2B2 gene or RNA and essentially does not hybridise to an ATP2B2 gene or RNA lacking said alteration. Specificity indicates that hybridisation to the target sequence generates a specific signal which can be distinguished from the signal generated through non-specific hybridisation. Perfectly complementary sequences are preferred to design probes according to this disclosure. It should be understood, however, that a certain degree of mismatch may be tolerated, as long as the specific signal may be distinguished from non-specific hybridisation.

Particular examples of such probes are nucleic acid sequences complementary to a target portion of the genomic region including the ATP2B2 gene or RNA carrying a point mutation as listed in Table 1 above. More particularly, the probes can comprise a sequence selected from the group consisting of SEQ ID Nos 3-10 or a fragment thereof comprising the SNP or a complementary sequence thereof.

The sequence of the probes can be derived from the sequences of the ATP2B2 gene and RNA as provided in the present application. Nucleotide substitutions may be performed, as well as chemical modifications of the probe. Such chemical modifications may be accomplished to increase the stability of hybrids (e.g., intercalating groups) or to label the probe. Typical examples of labels include, without limitation, radioactivity, fluorescence, luminescence, enzymatic labeling, etc.

The disclosure also concerns the use of a nucleic acid probe as described above in a method of detecting the presence of or predisposition to autism in a subject or in a method of assessing the response of a subject to a treatment of autism.

### Specific Ligand Binding

As indicated above, alteration in the ATP2B2 gene locus may also be detected by screening for alteration(s) in ATP2B2 polypeptide sequence or expression levels. In this regard, a specific embodiment of this invention comprises contacting the sample with a ligand specific for an ATP2B2 polypeptide and determining the formation of a complex.

Different types of ligands may be used, such as specific antibodies. In a specific embodiment, the sample is contacted with an antibody specific for an ATP2B2 polypeptide and the formation of an immune complex is determined. Various methods for detecting an immune complex can be used, such as ELISA, radioimmunoassays (RIA) and immuno-enzymatic assays (IEMA).

Within the context of this disclosure, an antibody designates a polyclonal antibody, a monoclonal antibody, as well as fragments or derivatives thereof having substantially the same antigen specificity. Fragments include Fab, Fab'2, CDR regions, etc. Derivatives include single-chain antibodies, humanized antibodies, poly-functional antibodies, etc.

An antibody specific for an ATP2B2 polypeptide designates an antibody that selectively binds an ATP2B2 polypeptide, namely, an antibody raised against an ATP2B2 polypeptide or an epitope-containing fragment thereof. Although non-specific binding towards other antigens may occur, binding to the target ATP2B2 polypeptide occurs with a higher affinity and can be reliably discriminated from non-specific binding.

In a specific embodiment, the method comprises contacting a sample from the subject with (a support coated with) an antibody specific for an altered form of an ATP2B2 polypeptide, and determining the presence of an immune complex. In a particular embodiment, the sample may be contacted simultaneously, or in parallel, or sequentially, with various (supports coated with) antibodies specific for different forms of an ATP2B2 polypeptide, such as a wild type and various altered forms thereof.

The disclosure also concerns the use of a ligand, preferably an antibody, a fragment or a derivative thereof as described above, in a method of detecting the presence of or predisposition to autism in a subject or in a method of assessing the response of a subject to a treatment of autism, an autism spectrum disorder, or an autism-associated disorder.

It is also described a diagnostic kit comprising products and reagents for detecting in a sample from a subject the presence of an alteration in the ATP2B2 gene or polypeptide, in the ATP2B2 gene or polypeptide expression, and/or in ATP2B2 activity. Said diagnostic kit comprises any primer, any pair of primers, any nucleic acid probe and/or any ligand, preferably antibody, described in the present invention. Said diagnostic kit according to the present disclosure can further comprise reagents and/or protocols for performing a hybridization, amplification or antigen-antibody immune reaction.

The diagnosis methods can be performed in vitro, ex vivo or in vivo, preferably in vitro or ex vivo. They use a sample from the subject, to assess the status of the ATP2B2 gene locus. The sample may be any biological sample derived from a subject, which contains nucleic acids or polypeptides. Examples of such samples include fluids, tissues, cell samples, organs, biopsies, etc. Most preferred samples are blood, plasma, saliva, urine, seminal fluid, etc. Pre-natal diagnosis may also be performed by testing fetal cells or placental cells, for instance. The sample may be collected according to conventional techniques and used directly for diagnosis or stored. The sample may be treated prior to performing the method, in order to render or improve availability of nucleic acids or polypeptides for testing. Treatments include, for instant, lysis (e.g., mechanical, physical, chemical, etc.), centrifugation, etc. Also, the nucleic acids and/or polypeptides may be pre-purified or enriched by conventional techniques, and/or reduced in complexity. Nucleic acids and polypeptides may also be treated with enzymes or other chemical or physical treatments to produce fragments thereof. Considering the high sensitivity of the claimed methods, very few amounts of sample are sufficient to perform the assay.

As indicated, the sample is preferably contacted with reagents such as probes, primers or ligands in order to assess the presence of an altered ATP2B2 gene locus. Contacting may be performed in any suitable device, such as a plate, tube, well, glass, etc. In specific embodiments, the contacting is performed on a substrate coated with the reagent, such as a nucleic acid array or a specific ligand array. The substrate may be a solid or semi-solid substrate such as any support comprising glass, plastic, nylon, paper, metal, polymers and the like. The substrate may be of various forms and sizes, such as a slide, a membrane, a bead, a column, a gel, etc. The contacting may be made under any condition suitable for a complex to be formed between the reagent and the nucleic acids or polypeptides of the sample.

The finding of an altered ATP2B2 polypeptide, RNA or DNA in the sample is indicative of the presence of an altered ATP2B2 gene locus in the subject, which can be correlated to the presence, predisposition or stage of progression of autism, an autism spectrum disorder, or an autism-associated disorder. For example, an individual having a germ line ATP2B2 mutation has an increased risk of developing autism. The determination of the presence of an altered ATP2B2 gene locus in a subject also allows the design of appropriate therapeutic intervention, which is more effective and customized. Also, this determination at the pre-symptomatic level allows a preventive regimen to be applied.

### Linkage Disequilibirum

Once a first SNP has been identified in a genomic region of interest, more particularly in ATP2B2 gene locus, the practitioner of ordinary skill in the art can easily identify additional SNPs in linkage disequilibrium with this first SNP. Indeed, any SNP in linkage disequilibrium with a first SNP associated with autism will be associated with this trait. Therefore, once the association has been demonstrated between a given SNP and autism , the discovery of additional SNPs associated with this trait can be of great interest in order to increase the density of SNPs in this particular region.

Identification of additional SNPs in linkage disequilibrium with a given SNP involves: (a) amplifying a fragment from the genomic region comprising or surrounding a first SNP from a plurality of individuals; (b) identifying of second SNPs in the genomic region harboring or surrounding said first SNP; (c) conducting a linkage disequilibrium analysis between said first SNP and second SNPs; and (d) selecting said second SNPs as being in linkage disequilibrium with said first marker. Subcombinations comprising steps (b) and (c) are also contemplated.

Methods to identify SNPs and to conduct linkage disequilibrium analysis can be carried out by the skilled person without undue experimentation by using well-known methods.

These SNPs in linkage disequilibrium can also be used in the methods according to the present invention, and more particularly in the diagnosic methods according to the present invention.

For example, a linkage locus of Crohn's disease has been mapped to a large region spanning 18cM on chromosome 5q31 (Rioux et al., 2000 and 2001). Using dense maps of microsatellite markers and SNPs across the entire region, strong evidence of linkage disequilibrium (LD) was found. Having found evidence of LD, the authors developed an ultra-high-density SNP map and studied a denser collection of markers selected from this map. Multilocus analyses defined a single common risk haplotype characterised by multiple SNPs that were each independently associated using TDT. These SNPs were unique to the risk haplotype and essentially identical in their information content by virtue of being in nearly complete LD with one another. The equivalent properties of these SNPs make it impossible to identify the causal mutation within this region on the basis of genetic evidence alone.

### Causal Mutation

Mutations in the ATP2B2 gene which are responsible for autism may be identified by comparing the sequences of the ATP2B2 gene from patients presenting autism and control individuals. Based on the identified association of SNPs of ATP2B2 and autism, the identified locus can be scanned for mutations. In a preferred embodiment, functional regions such as exons and splice sites, promoters and other regulatory regions of the ATP2B2 gene are scanned for mutations. Preferably, patients presenting autism carry the mutation shown to be associated with and controls individuals do not carry the mutation or allele associated with disorder. It might also be possible that patients presenting autism carry the mutation shown to be associated with autism with a higher frequency than controls individuals.

The method used to detect such mutations generally comprises the following steps: amplification of a region of the ATP2B2 gene comprising a SNP or a group of SNPs associated with autism from DNA samples of the ATP2B2 gene from patients presenting autism and control individuals; sequencing of the amplified region; comparison of DNA sequences of the ATP2B2 gene from patients presenting autism and control individuals; determination of mutations specific to patients presenting autism.

Therefore, identification of a causal mutation in the ATP2B2 gene can be carried out by the skilled person without undue experimentation by using well-known methods.

For example, the causal mutations have been identified in the following examples by using routine methods.

Hugot et al. (2001) applied a positional cloning strategy to identify gene variants with susceptibly to Crohn's disease in a region of chromosome 16 previously found to be linked to susceptibility to Crohn's disease. To refine the location of the potential sucecptibility locus 26 microsatellite markers were genotyped and tested for association to Crohn's disease using the transmission disequilibrium test. A borderline significant association was found between one allele of the microsatellite marker D16S136. Eleven additional SNPs were selected from surrounding regions and several SNPs showed significant association. SNP5-8 from this region were found to be present in a single exon of the NOD2/CARD15 gene and shown to be non-synonymous variants. This prompted the authors to sequence the complete coding sequence of this gene in 50 CD patients. Two additional non-synonymous mutations (SNP12 and SNP13) were found. SNP13 was most significant associated (p=6x10-6) using the pedigree transmission disequilibrium test. In another independent study, the same variant was found also by sequencing the coding region of this gene from 12 affected individuals compared to 4 controls (Ogura et al., 2001). The rare allele of SNP13 corresponded to a 1-bp insertion predicted to truncate the NOD2/CARD15 protein. This allele was also present in normal healthy individuals, albeit with significantly lower frequency as compared to the controls.

Similarly, Lesage et al. (2002) performed a mutational analyses of CARD 15 in 453 patients with CD, including 166 sporadic and 287 familial cases, 159 patients with ulcerative colitis (UC), and 103 healthy control subjects by systematic sequencing of the coding region. Of 67 sequence variations identified, 9 had an allele frequency >5% in patients with CD. Six of them were considered to be polymorphisms, and three (SNP12-R702W, SNP8-G908R, and SNP13-1007fs) were confirmed to be independently associated with susceptibility to CD. Also considered as potential disease-causing mutations (DCMs) were 27 rare additional mutations. The three main variants (R702W, G908R, and 1007fs) represented 32%, 18%, and 31%, respectively, of the total CD mutations, whereas the total of the 27 rare mutations represented 19% of DCMs. Altogether, 93% of the mutations were located in the distal third of the gene. No mutations were found to be associated with UC. In contrast, 50% of patients with CD carried at least one DCM, including 17% who had a double mutation.

### DRUG SCREENING

The present invention also provides novel targets and methods for the screening of drug candidates or leads. The methods include binding assays and/or functional assays, and may be performed in vitro, in cell systems, in animals, etc.

A particular object of this invention resides in a method of selecting biologically active compounds, said method comprising contacting in vitro a test compound with an ATP2B2 gene or polypeptide according to the present invention and determining the ability of said test compound to bind said ATP2B2 gene or polypeptide. Binding to said gene or polypeptide provides an indication as to the ability of the compound to modulate the activity of said target, and thus to affect a pathway leading to autism in a subject. In a preferred embodiment, the method comprises contacting in vitro a test compound with an ATP2B2 polypeptide or a fragment thereof according to the present invention and determining the ability of said test compound to bind said ATP2B2 polypeptide or fragment. The fragment preferably comprises a binding site of the ATP2B2 polypeptide. Preferably, said ATP2B2 gene or polypeptide or a fragment thereof is an altered or mutated ATP2B2 gene or polypeptide or a fragment thereof comprising the alteration or mutation.

A particular object of this invention resides in a method of selecting compounds active on autism, said method comprising contacting in vitro a test compound with an ATP2B2 polypeptide according to the present invention or binding site-containing fragment thereof and determining the ability of said test compound to bind said ATP2B2 polypeptide or fragment thereof. Preferably, said ATP2B2 polypeptide or a fragment thereof is an altered or mutated ATP2B2 polypeptide or a fragment thereof comprising the alteration or mutation.

In a further particular embodiment, the method comprises contacting a recombinant host cell expressing an ATP2B2 polypeptide according to the present invention with a test compound, and determining the ability of said test compound to bind said ATP2B2 and to modulate the activity of ATP2B2 polypeptide. Preferably, said ATP2B2 polypeptide or a fragment thereof is an altered or mutated ATP2B2 polypeptide or a fragment thereof comprising the alteration or mutation.

The determination of binding may be performed by various techniques, such as by labeling of the test compound, by competition with a labeled reference ligand, etc.

A further object of this invention resides in a method of selecting biologically active compounds, said method comprising contacting in vitro a test compound with an ATP2B2 polypeptide according to the present invention and determining the ability of said test compound to modulate the activity of said ATP2B2 polypeptide. Preferably, said ATP2B2 polypeptide or a fragment thereof is an altered or mutated ATP2B2 polypeptide or a fragment thereof comprising the alteration or mutation.

A further object of this invention resides in a method of selecting biologically active compounds, said method comprising contacting in vitro a test compound with an ATP2B2 gene according to the present invention and determining the ability of said test compound to modulate the expression of said ATP2B2 gene. Preferably, said ATP2B2 gene or a fragment thereof is an altered or mutated ATP2B2 gene or a fragment thereof comprising the alteration or mutation.

In an other embodiment, this invention relates to a method of screening, selecting or identifying active compounds, particularly compounds active on autism , the method comprising contacting a test compound with a recombinant host cell comprising a reporter construct, said reporter construct comprising a reporter gene under the control of an ATP2B2 gene promoter, and selecting the test compounds that modulate (e.g. stimulate or reduce) expression of the reporter gene. Preferably, said ATP2B2 gene promoter or a fragment thereof is an altered or mutated ATP2B2 gene promoter or a fragment thereof comprising the alteration or mutation.

In a particular embodiment of the methods of screening, the modulation is an inhibition. In another particular embodiment of the methods of screening, the modulation is an activation.

The above screening assays may be performed in any suitable device, such as plates, tubes, dishes, flasks, etc. Typically, the assay is performed in multi-wells plates. Several test compounds can be assayed in parallel. Furthermore, the test compound may be of various origin, nature and composition. It may be any organic or inorganic substance, such as a lipid, peptide, polypeptide, nucleic acid, small molecule, etc., in isolated or in mixture with other substances. The compounds may be all or part of a combinatorial library of products, for instance.

Further aspects and advantages of the present invention will be disclosed in the following experimental section, which should be regarded as illustrative and not limiting the scope of the present application.

### GENE, VECTORS, RECOMBINANT CELLS AND POLYPEPTIDES

Here are described products for use in diagnosis, or screening. These products comprise nucleic acid molecules encoding an ATP2B2 polypeptide or a fragment thereof, vectors comprising the same, recombinant host cells and expressed polypeptides.

More particularly, it is described an altered or mutated ATP2B2 gene or a fragment thereof comprising said alteration or mutation. Further disclosed are nucleic acid molecules encoding an altered or mutated ATP2B2 polypeptide or a fragment thereof comprising said alteration or mutation. Said alteration or mutation modifies the ATP2B2 activity. The modified activity can be increased or decreased. It is further described a vector comprising an altered or mutated ATP2B2 gene or a fragment thereof comprising said alteration or mutation or a nucleic acid molecule encoding an altered or mutated ATP2B2 polypeptide or a fragment thereof comprising said alteration or mutation, recombinant host cells and expressed polypeptides.

It is further described a vector comprising a nucleic acid encoding an ATP2B2 polypeptide as herein defined. The vector may be a cloning vector or, more preferably, an expression vector, i.e., a vector comprising regulatory sequences causing expression of an ATP2B2 polypeptide from said vector in a competent host cell.

These vectors can be used to express an ATP2B2 polypeptide in vitro, ex vivo or in vivo, to create transgenic or "Knock Out" non-human animals, to amplify the nucleic acids, to express antisense RNAs, etc.

The vectors described herein typically comprise an ATP2B2 coding sequence according to the present invention operably linked to regulatory sequences, e.g., a promoter, a polyA, etc. The term "operably linked" indicates that the coding and regulatory sequences are functionally associated so that the regulatory sequences cause expression (e.g., transcription) of the coding sequences. The vectors may further comprise one or several origins of replication and/or selectable markers. The promoter region may be homologous or heterologous with respect to the coding sequence, and may provide for ubiquitous, constitutive, regulated and/or tissue specific expression, in any appropriate host cell, including for in vivo use. Examples of promoters include bacterial promoters (T7, pTAC, Trp promoter, etc.), viral promoters (LTR, TK, CMV-IE, etc.), mammalian gene promoters (albumin, PGK, etc), and the like.

The vector may be a plasmid, a virus, a cosmid, a phage, a BAC, a YAC, etc. Plasmid vectors may be prepared from commercially available vectors such as pBluescript, pUC, pBR, etc. Viral vectors may be produced from baculoviruses, retroviruses, adenoviruses, AAVs, etc., according to recombinant DNA techniques known in the art.

In this regard, it is described a recombinant virus encoding an ATP2B2 polypeptide as defined above. The recombinant virus is preferably replication-defective, even more preferably selected from E1- and/or E4-defective adenoviruses, Gag-, pol- and/or env-defective retroviruses and Rep- and/or Cap-defective AAVs. Such recombinant viruses may be produced by techniques known in the art, such as by transfecting packaging cells or by transient transfection with helper plasmids or viruses. Typical examples of virus packaging cells include PA317 cells, PsiCRIP cells, GPenv+ cells, 293 cells, etc. Detailed protocols for producing such replication-defective recombinant viruses may be found for instance in WO95/14785, WO96/22378, US5,882,877, US6,013,516, US4,861,719, US5,278,056 and WO94/19478.

A further object of the present disclosure resides in a recombinant host cell comprising a recombinant ATP2B2 gene or a vector as defined above. Suitable host cells include, without limitation, prokaryotic cells (such as bacteria) and eukaryotic cells (such as yeast cells, mammalian cells, insect cells, plant cells, etc.). Specific examples include E. coli, Kluyveromyces or Saccharomyces yeasts, mammalian cell lines (e.g., Vero cells, CHO cells, 3T3 cells, COS cells, etc.) as well as primary or established mammalian cell cultures (e.g., produced from fibroblasts, embryonic cells, epithelial cells, nervous cells, adipocytes, etc.).

It is further described a method for producing a recombinant host cell expressing an ATP2B2 polypeptide as defined herein, said method comprising (i) introducing in vitro or ex vivo into a competent host cell a recombinant nucleic acid or a vector as described above, (ii) culturing in vitro or ex vivo the recombinant host cells obtained and (iii), optionally, selecting the cells which express the ATP2B2 polypeptide.

Such recombinant host cells can be used for the production of ATP2B2 polypeptides, as well as for screening of active molecules, as described below. Such cells may also be used as a model system to study autism. These cells can be maintained in suitable culture media, such as DMEM, RPMI, HAM, etc., in any appropriate culture device (plate, flask, dish, tube, pouch, etc.).

### EXAMPLES

### 1. GenomeHIP platform to identify the chromosome 3 susceptibility gene

The GenomeHIP platform was applied to allow rapid identification of an autism susceptibility gene.

Briefly, the technology consists of forming pairs from the DNA of related individuals. Each DNA is marked with a specific label allowing its identification. Hybrids are then formed between the two DNAs. A particular process (WO00/53802) is then applied that selects all fragments identical-by-descent (IBD) from the two DNAs in a multi step procedure. The remaining IBD enriched DNA is then scored against a BAC clone derived DNA microarray that allows the positioning of the IBD fraction on a chromosome.

The application of this process over many different families results in a matrix of IBD fractions for each pair from each family. Statistical analyses then calculate the minimal IBD regions that are shared between all families tested. Significant results (p-values) are evidence for linkage of the positive region with the trait of interest (here autism). The linked interval can be delimited by the two most distant clones showing significant p-values.

In the present study, 114 families from the United States (114 independent sib-pairs) concordant for strict autism (as defined by ADI-R) were submitted to the GenomeHIP process. The resulting IBD enriched DNA fractions were then labeled with Cy5 fluorescent dyes and hybridised against a DNA array consisting of 2263 BAC clones covering the whole human genome with an average spacing of 1.2 Mega base pairs. Non-selected DNA labeled with Cy3 was used to normalize the signal values and compute ratios for each clone. Clustering of the ratio results was then performed to determine the IBD status for each clone and pair.

By applying this procedure, a BAC clone was identified (FE0DBACAI7ZG05v) which showed suggestive evidence for linkage to autism (p=6.4e-05). The linkage region was spanning approximately 2.18 megabases in the region on chromosome 3 (bases 9283670 to 11464577) as defined by the clones proximal and distal of the BAC clone showing suggestive evidence for linkage. The p-value of 7.4E-04 was used for suggestive evidence for linkage as proposed by Kruglyak and Lander (1995) for whole genome scans in complex traits.

Table 2: Linkage results for chromosome 3 in the ATP2B2 locus: Indicated is the region corresponding to the BAC clone with evidence for linkage. The start and stop positions of the clones correspond to their genomic locations based on NCBI Build34 with respect to the start of the chromosome (p-ter).

**Table 2**

| Human chromosome | Clone | Start | Stop | Number of informative pairs | p-value |
|---|---|---|---|---|---|
| 3 | FE0DBACA2ZH12v | 9124735 | 9283670 | 81 | 0.004 |
| 3 | FE0DBACA17ZG05v | 9721553 | 9931071 | 104 | 6.4e-05 |
| 3 | FEODBACA18ZE05v | 11464577 | 11591404 | 77 | 0.017 |

### 2. Identification of an autism susceptibility gene on chromosome 3

By screening the aforementioned 2.18 Megabases in the linked chromosomal region, we identified the ATPase, Ca++ transporting, plasma membrane 2 as a candidate for autism and related phenotypes. This gene is indeed present in the critical interval, with evidence for linkage delimited by the clones outlined above.

The ATP2B2 gene encodes a predicted 1243-amino acid polypeptide for isoform a for NP_001001331, (mRNA NM_001001331, 6821 bp) and spreads over 380 kb of genomic sequence. Alternatively spliced transcript variants encoding different isoforms have been identified for this gene. The protein encoded by this gene is a member of the cation transport ATPase (P-type) family, type IIB subfamily and is characterized by the formation of an aspartyl phosphate intermediate during the reaction cycle. These enzymes remove bivalent calcium ions from eukaryotic cells against very large concentration gradients and play a critical role in intracellular calcium homeostasis.

Zaccharias et al (1997) employed in situ hybridization to determine the expression pattern of the four human PMCA isoforms in the human hippocampus. PMCA1 and 3 mRNAs were weakly expressed throughout the hippocampal formation, whereas PMCA2 and 4 mRNA expression showed distinct regional differences, with increased levels in CA2 and the dentate gyrus.

To analyze the physiologic role of PMCA2, Kozel et al. (1998) produced PMCA2-deficient mice by gene targeting. Homozygous PMCA2-null mice grew more slowly than heterozygous and wildtype mice and exhibited an unsteady gait and difficulties in maintaining balance. Histologic analysis of the cerebellum and inner ear of mutant and wildtype mice showed that null mutants have slightly increased numbers of Purkinje neurons (in which PMCA2 is highly expressed), a decreased thickness of the molecular layer, an absence of otoconia in the vestibular system, and a range of abnormalities of the organ of Corti. Analysis of auditory-evoked brain stem responses showed that homozygous mutants were deaf and that heterozygous mice had a significant hearing loss. These data demonstrated that PMCA2 is required for both balance and hearing and suggested that it may be a major source of the calcium used in the formation and maintenance of otoconia.

Street et al. (1998) reported that the gene encoding a plasma membrane Ca2+-ATPase type 2 pump (ATP2B2, also known as PMCA2) is mutated in dfw. An A-->G nucleotide transition in dfw DNA causes a glycine-to-serine substitution at a highly conserved aminoacid position, whereas in a second allele, dfw2J, a 2-base-pair deletion causes a frameshift that predicts a truncated protein. In the cochlea, the protein ATP2B2 is localized to stereocilia and the basolateral wall of hair cells in wild-type mice, but is not detected in dfw2J mice. This indicates that mutation of ATP2B2 may cause deafness and imbalance by affecting sensory transduction in stereocilia as well as neurotransmitter release from the basolateral membrane.

Ueno et al. (2002) identified mice with a nucleotide transition in the PCMA2 gene which caused a glutamic acid to change into lysine. The mice showed behavioral defects such as severe tremor, up-and-down and side-to-side wriggling of neck without coordination. Since PMCA2 is expressed in the cerebellum and plays an important role to maintain the homeostasis of the intracellular Ca2+ as a Ca2+ pump, the behavioral defect can be ascribed to the impairment of Ca2+ regulation in neurons of the cerebellum. To confirm the defect of Ca2+ homeostasis in the mutant mice, high K+-induced changes were measured in intracellular Ca2+ concentration ([Ca2+]i) in the cerebellar neurons. The rate of rise in [Ca2+]i during high K+-induced depolarization was significantly reduced, and the extrusion rate of increased [Ca2+]i was also reduced. These results suggested that voltage-gated Ca2+ channels were down-regulated in the mutant mice in order to regulate [Ca2+]i toward the normal homeostasis. The behavioral defects may be ascribed to the down-regulated Ca2+ homeostasis since dynamic changes in [Ca2+]i are important for various neuronal functions.

Kozel et al. (2002) hypothesized that PMCA2 may be the first gene with a known mutated protein product that confers increased susceptibility to noise induced hearing loss.

Pronounced to profound bilateral hearing loss or deafness was diagnosed in cases of autistic disorders, representing a prevalence considerably above that in the general population and comparable to the prevalence found in populations with mental retardation (Rosenhall, et al. 1999). Mild to moderate hearing loss was diagnosed in 7.9% and unilateral hearing loss in 1.6% of those who could be tested appropriately. Hearing deficits in autism occurred at similar rates at all levels of intellectual functioning, so it does not appear that the covariation with intellectual impairment per se can account for all of the variance of hearing deficit in autism. Hyperacusis was common, affecting 18.0% of the autism group and 0% in an age-matched nonautism comparison group. In addition, the rate of serous otitis media (23.5%) and related conductive hearing loss (18.3%) appeared to be increased in autistic disorder.

Recent findings in autistic children and adults reported by Boddaert et al. (2003, 2004) suggest that the inadequate behavioral responses to sounds and the language impairments typically seen in autism could be due to abnormal auditory cortical processing.

Findings by Kurnellas et al. (2005) suggest that a reduction in PMCA2 level or activity leading to delays in calcium clearance may cause neuronal damage and loss in the spinal cord.

Taken together, the linkage results provided in the present application, identifying the human ATP2B2 gene in the critical interval of genetic alterations linked to autism on chromosome 3, with its involvement in neuronal function and hearing loss, we conclude that alterations (e.g., mutations and/or polymorphisms) in the ATP2B2 gene or its regulatory sequences may contribute to the development of human autism and represent a novel target for diagnosis or therapeutic intervention.

### 3. Association study

The same families that have been used for the linkage study were also used to test for association between a specific phenotype (here autism) in question and the genetic marker allele or haplotypes containing a specific marker allele using the transmission disequilibrium test (TDT). The TDT is a powerful association test as it is insensitive to population stratification problems in the tested sample. Briefly, the segregation of alleles from heterozygous parents to their affected offspring is tested. The portion of alleles transmitted to the affected offspring compared to the non-transmitted alleles is compared to the ratio expected under random distribution. A significant excess of allele transmission over the expected value is evidence for an association of the respective allele or haplotype with the studied autism phenotype.

The results of this analysis show that certain alleles of the ATP2B2 gene are positively associated with autism and therefore increase the susceptibility to disease. In the tested population, the allele 1 (A) of SNP22 is correlated with autism as determined by TDT (p-value = 0.0476). In contrast, the allele 2 (G) of SNP22 is significantly under-transmitted to autistic individuals showing that this allele helps protect from the disease.

Examples of the transmission of the alleles to autists are given in Table 3.

**Table 3**

| SNP | Allele | Allele transmitted to individuals (N) | Allele not transmitted to individuals (N) | p-value |
|---|---|---|---|---|
| SNP22 | 1 | 79 | 56 | 0.0476 |
| SNP22 | 2 | 56 | 79 | 0.0476 |

In addition, haplotypes were constructed for SNP21, SNP22, SNP28, SNP39, SNP46, SNP61, SNP73 and SNP74 to identify the phase for all SNPs.

The results of this analysis in the tested population showed that certain haplotypes, all characterized by the presence of allele 2 (T) at SNP21, allele 1 (A) at SNP22 or allele 2 (T) at SNP28 are significantly associated with autism, while certain haplotypes devoid of the alleles 2 (T), 1 (A) or 2 (T), respectively, are preferentially not transmitted to autists. An example is the haplotype 2-1 (T-C) for SNP21-SNP46, p = 0.001644. Haplotypes that carry allele 1 (C) instead of allele 2 (T) at SNP21 or allele 1 (C) instead of allele 2 (T) at SNP 28 show significant evidence to be under-represented in autistic subjects. An example is the haplotype 1-1 (C-A) for SNP28-SNP61, p = 0.008087.

Examples of haplotypes with preferential transmission and non-transmission to autists are given in Table 4.

**Table 4**

| SNPs used to construct haplotype | Alleles composing haplotype | Frequency of haplotype transmitted to autists | Frequency of haplotype not transmitted to autists | p-value |
|---|---|---|---|---|
| 21-46 | 1-2 | 0.3436 | 0.4454 | 0.05121 |
| 21-46 | 2-1 | 0.2054 | 0.06262 | 0.001644 |
| 21-73 | 2-2 | 0.257 | 0.1487 | 0.01614 |
| 21-74 | 2-1 | 0.3023 | 0.1683 | 0.00429 |
| 22-28 | 1-2 | 0.3589 | 0.2258 | 0.00798 |
| 22-39 | 1-2 | 0.5072 | 0.3582 | 0.008166 |
| 28-61 | 1-1 | 0.2793 | 0.4239 | 0.008087 |
| 28-61 | 2-1 | 0.4417 | 0.3203 | 0.03212 |

### REFERENCES

Asperger (1944) Die autistischen Psychopathen im Kindesalter. Archiv für Psychiatrie und Nervenkrankheiten, 2:217-250.
Bailey A, Le Couteur A, Gottesman I, et al. (1995) Autism as a strongly genetic disorder: evidence from a British twin study. Psychol Med, 25:63-77.
Bailey A, Phillips W, Rutter M (1996) Autism: towards an integration of clinical, genetic, neuropsychological, and neurobiological perspectives. J Child Psychol Psychiatry 37(1):89-126.
Baird G, Charman T, Baron-Cohen S et al. (2000) A screening instrument for autism at 18 months of age: a 6-year follow-up study. J Am Acad Child Adolesc Psychiatry, 39(6):694-702.
Boddaert N, Belin P, Chabane N et al. (2003) Perception of complex sounds: abnormal pattern of cortical activation in autism. Am J Psychiatry, 160(11):2057-2060.
Boddaert N, Chabane N, Belin P et al. (2004) Perception of complex sounds in autism: abnormal auditory cortical processing in children. Am J Psychiatry, 161(11):2117-2120.
Burger R and Warren R (1998) Possible immunogenetic basis for autism. Ment Retard Dev Disabil Res Rev, 4:137-141.
Carney RM, Wolpert CM, Ravan SA et al. (2003) Identification of MeCP2 mutations in a series of females with autistic disorder. Pediatr Neurol, 28(3):205-211.
Chakrabarti S and Fombonne E (2001) Pervasive developmental disorders in preschool children. JAMA, 285(24):3093-9
Comi AM, Zimmerman AW, Frye VH et al. (1999) Familial clustering of autoimmune disorders and evaluation of medical risk factors in autism. J Child Neurol, 14(6):388-394.
Connolly AM, Chez MG, Pestronk A et al. (1999) Serum autoantibodies to brain in Landau-Kleffner variant, autism, and other neurologic disorders. J Pediatr, 134(5):607-613.
Folstein S and Rutter M (1977) Infantile autism: a genetic study of 21 twin pairs. J Child Psychol Psychiatry Allied Disciplines, 18:297-321.
Folstein SE and Rosen-Sheidley BR (2001) Genetics of autism: complex aetiology for a heterogeneous disorder. Nat Rev Genet, 2:943-955.
Gillberg C (1998) Chromosomal disorders and autism. J Autism Dev Disord, 28(5):415-425.
Gillberg C and Coleman M (2000) The biology of the autistic syndromes, 3rd edn London: MacKeith Press.
Gillberg C and Wing L (1999) Autism: not an extremely rare disorder. Acta Psychiatr Scand, 99:339-406.
Hugot JP, Chamaillard M, Zouali H et al. (2001) Association of NOD2 leucine-rich repeat variants with susceptibility to Crohn's disease. Nature 411(6837):599-603.
Jamain S, Quach H, Betancur C et al. (2003) Mutations of the X-linked genes encoding neuroligins NLGN3 and NLGN4 are associated with autism. Nat Genet, 34(1):27-29.
Jorde LB, Hasstedt SJ, Ritvo ER et al. (1991) Complex segregation analysis of autism. Am J Hum Genet, 49(5):932-938.
Jorde LB, Mason-Brothers A, Waldmann R et al. (1990) The UCLA-University of Utah epidemiologic survey of autism: genealogical analysis of familial aggregation. Am J Med Genet, 36(1):85-88.
Kanner L (1943) Autistic disturbances of affective contact. Nervous Child, 2:217-250.
Kozel PJ, Friedman RA, Erway LC et al. (1998) Balance and hearing deficits in mice with a null mutation in the gene encoding plasma membrane Ca2+-ATPase isoform 2. J Biol Chem, 273(30):18693-18696.
Kozel PJ, Davis RR, Krieg EF et al. (2002) Deficiency in plasma membrane calcium ATPase isoform 2 increases susceptibility to noise-induced hearing loss in mice. Hear Res, 164(1-2):231-239.
Kurnellas MP, Nicot A, Shull GE, Elkabes S (2005) Plasma membrane calcium ATPase deficiency causes neuronal pathology in the spinal cord: a potential mechanism for neurodegeneration in multiple sclerosis and spinal cord injury. FASEB J, 19(2):298-300.
Lander E and Kruglyak L (1995) Genetic dissection of complex traits: guidelines for interpreting and reporting linkage results. Nat Genet, 11(3):241-247.
Le Couteur A, Rutter M, Lord C et al. (1989) Autism diagnostic interview: a standardized investigator-based instrument. J Autism Dev Disord, 19(3):363-387.
Lesage S, Zouali H, Cezard Jpet al. (2002) CARD15/NOD2 mutational analysis and genotype-phenotype correlation in 612 patients with inflammatory bowel disease. Am J Hum Genet. 70(4):845-857.
Lord C, Rutter M, Le Couteur A (1994) Autism Diagnostic Interview-Revised: a revised version of a diagnostic interview for caregivers of individuals with possible pervasive developmental disorders. J Autism Dev Disord, 24(5):659-685.
Nelson KB (1991) Prenatal and perinatal factors in the etiology of autism. Pediatrics, 87(5 Pt 2):761-766.
Ogura Y, Bonen DK, Inohara N (2001) A framshift mutation in NOD2 associated with susceptibility to Crohn's disease. Nature 411(6837):603-606.
Rioux JD, Daly MJ, Silverberg MS et al. (2001) Genetic variation in the 5q31 cytokine gene cluster confers susceptibility to Crohn disease. Nat Genet 29(2): 223-228.
Rioux JD, Silverberg MS, Daly MJ (2000) Genomewide search in Canadian families with inflammatory bowel disease reveals two novel susceptibility loci. Am J Hum Genet 66(6):1863-1870.
Rodier P and Hyman S (1998) Early environmental factors in autism. Mental Retard Dev Disord Res Rev, 4:121-128.
Rosenhall U, Nordin V, Sandstrom M et al. (1999) Autism and hearing loss. J Autism Dev Disord 29(5):349-357.
Singh VK, Warren RP, Odell JD et al. (1993) Antibodies to myelin basic protein in children with autistic behavior. Brain Behav Immun, 7(1):97-103.
Smalley SL (1997) Genetic influences in childhood-onset psychiatric disorders: autism and attention-deficit/hyperactivity disorder. Am J Hum Genet, 60(6):1276-1282.
Steffenburg S, Gillberg C, Hellgren L et al. (1989) A twin study of autism in Denmark, Finland, Iceland, Norway and Sweden. J Child Psychol Psychiatry, 30(3):405-416.
Street VA, McKee-Johnson JW, Fonseca RC et al. (1998) Mutations in a plasma membrane Ca2+-ATPase gene cause deafness in deafwaddler mice. Nat Genet, 19(4):390-394. Szatmari P, Jones MB, Zwaigenbaum L et al. (1998) Genetics of autism: overview and new directions. J Autism Dev Disord, 28(5):351-368.
Ueno T, Kameyama K, Hirata M et al. (2002) A mouse with a point mutation in plasma membrane Ca2+-ATPase isoform 2 gene showed the reduced Ca2+ influx in cerebellar neurons. Neurosci Res, 42(4):287-297.
Weizman A, Weizman R, Szekely GA et al. (1982) Abnormal immune response to brain tissue antigen in the syndrome of autism. Am J Psychiatry, 139(11):1462-1465.
Zacharias DA, DeMarco SJ, Strehler EE (1997) mRNA expression of the four isoforms of the human plasma membrane Ca(2+)-ATPase in the human hippocampus. Brain Res Mol Brain Res, 45(1):173-176.

## Claims

1. An *in vitro* method of detecting the presence of or predisposition to autism in a subject, the method comprising detecting the presence of an alteration in the ATP2B2 gene in a sample from the subject.

2. An *in vitro* method of detecting the protection from autism in a subject, the method comprising detecting the presence of an alteration in the ATP2B2 gene in a sample from the subject.

3. The method of any one of claims 1-2, wherein the presence of an alteration in the ATP2B2 gene is detected by sequencing, selective hybridisation and/or selective amplification.

4. The method of any one of claims 1-2, wherein said alteration is one or several SNP(s) or a haplotype of SNPs associated with autism.

5. The method of claim 4, wherein said haplotype associated with autism comprises several SNPs selected from the group consisting of SNP21 (SEQ ID NO:3), SNP22 (SEQ ID NO:4), SNP28 (SEQ ID NO:5), SNP39 (SEQ ID NO:6), SNP46 (SEQ ID NO:7), SNP61 (SEQ ID NO:8), SNP73 (SEQ ID NO:9) and SNP74 (SEQ ID NO: 10).

6. The method of claim 4, wherein said SNP associated with autism is SNP22.

7. A method of selecting biologically active compounds on autism, said method comprising contacting a test compound with an ATP2B2 polypeptide or gene or a fragment thereof and determining the ability of said test compound to bind the ATP2B2 polypeptide or gene or a fragment thereof.

8. A method of selecting biologically active compounds on autism, said method comprising contacting a recombinant host cell expressing an ATP2B2 polypeptide with a test compound, and determining the ability of said test compound to bind said ATP2B2 polypeptide and to modulate the activity of ATP2B2 polypeptide.

9. A method of selecting biologically active compounds on autism, said method comprising contacting a test compound with an ATP2B2 gene and determining the ability of said test compound to modulate the expression of said ATP2B2 gene.

10. A method of selecting biologically active compounds on autism, said method comprising contacting a test compound with a recombinant host cell comprising a reporter construct, said reporter construct comprising a reporter gene under the control of an ATP2B2 gene promoter, and selecting the test compounds that modulate (e.g. stimulate or reduce) expression of the reporter gene.

11. Method according any one of claims 7-10, wherein said ATP2B2 gene or polypeptide or a fragment thereof is an altered or mutated ATP2B2 gene or polypeptide or a fragment thereof comprising the alteration or mutation.

12. Method according any one of claims 7-11, wherein said modulation is an activation.

13. Method according any one of claims 7-11, wherein said modulation is an inhibition.

## Patentansprüche

1. Ein *in vitro* Verfahren zum Nachweis des Vorhandenseins von oder der Veranlagung zu Autismus in einem Patienten, wobei das Verfahren den Nachweis des Vorhandenseins einer Änderung in dem ATP2B2 Gen in einer Probe eines Patienten umfasst.

2. Ein *in vitro* Verfahren zum Nachweis des Schutzes vor Autismus in einem Patienten, wobei das Verfahren den Nachweis des Vorhandenseins einer Änderung in dem ATP2B2 Gen in einer Probe eines Patienten umfasst.

3. Das Verfahren nach einem der Ansprüche 1 bis 2, wobei die Anwesenheit einer Änderung in dem ATP2B2 Gen durch Sequenzieren, selektive Hybridisierung und/oder selektive Amplifikation nachgewiesen wird.

4. Das Verfahren nach einem der Ansprüche 1 bis 2, wobei die Änderung ein oder mehrere SNP(s) oder ein mit Autismus assoziierter Haplotyp der SNPs ist.

5. Das Verfahren nach Anspruch 4, wobei der mit Autismus assoziierter Haplotyp mehrere SNPs ausgewählt aus der Gruppe bestehend aus SNP21 (SEQ ID NO: 3), SNP22 (SEQ ID NO: 4), SNP28 (SEQ ID NO: 5), SNP39 (SEQ ID NO: 6), SNP46 (SEQ ID NO: 7), SNP61 (SEQ ID NO: 8), SNP73 (SEQ ID NO: 9) und SNP74 (SEQ ID NO: 10) umfasst.

6. Das Verfahren nach Anspruch 4, wobei der mit Autismus assoziierter SNP SNP22 ist.

7. Ein Verfahren zum Auswählen biologisch wirksamer Verbindungen gegen Autismus, wobei das Verfahren das in Kontakt bringen einer Testverbindung mit einem ATP2B2 Polypeptid oder einem Gen oder einem Fragment davon und das Bestimmen der Fähigkeit dieser Testverbindung das ATP2B2 Polypeptid oder Gen oder ein Fragment davon zu binden, umfasst.

8. Ein Verfahren zum Auswählen biologisch wirksamer Verbindungen gegen Autismus, wobei das Verfahren das in Kontakt bringen einer rekombinanten Wirtszelle, die ein ATP2B2 Polypeptid exprimiert, mit einer Testverbindung und das Bestimmen der Fähigkeit der Testverbindung das ATP2B2 Polypeptid zu binden und die Aktivität des ATP2B2 Polypeptids zu modulieren, umfasst.

9. Ein Verfahren zum Auswählen biologisch wirksamer Verbindungen gegen Autismus, wobei das Verfahren das in Kontakt bringen einer Testverbindung mit einem ATP2B2 Polypeptid und das Bestimmen der Fähigkeit der Testverbindung die Expression des ATP2B2 Gens zu modulieren, umfasst.

10. Ein Verfahren zum Auswählen biologisch wirksamer Verbindungen gegen Autismus, wobei das Verfahren das in Kontakt bringen einer Testverbindung mit einer rekombinanten Wirtszelle umfassend ein Reporter Konstrukt, wobei das Reporter-Konstrukt ein Reporter-Gen unter der Kontrolle eines ATP2B2-Promotors umfasst und das Auswählen der Testverbindungen, die die Expression des Reporter-Gens modulieren (z.B. stimulieren oder reduzieren), umfasst.

11. Verfahren nach einem der Ansprüche 7-10, wobei das ATP2B2 Gen oder Polypeptid oder ein Fragment davon ein geändertes oder mutiertes ATP2B2 Gen oder Polypeptid oder Fragment davon ist, umfassend die Änderung oder Mutation.

12. Verfahren nach einem der Ansprüche 7-11, wobei es sich bei der Modulation um eine Aktivierung handelt.

13. Verfahren nach einem der Ansprüche 7-11, wobei es sich bei der Modulation um eine Inhibierung handelt.

## Revendications

1. Méthode *in vitro* de détection de la présence de ou de la prédisposition à l'autisme chez un sujet, la méthode comprenant la détection de la présence d'une altération dans le gène ATP2B2 dans un échantillon du sujet.

2. Méthode *in vitro* de détection de la protection contre l'autisme chez un sujet, la méthode comprenant la détection de la présence d'une altération dans le gène ATP2B2 dans un échantillon du sujet.

3. Méthode selon l'une quelconque des revendications 1-2, dans laquelle la présence d'une altération dans le gène ATP2B2 est détectée par un séquençage, une hybridation sélective et/ou une amplification sélective.

4. Méthode selon l'une quelconque des revendications 1-2, dans laquelle ladite altération est un ou plusieurs SNP(s) ou un halotype de SNPs associé à l'autisme.

5. Méthode de la revendication 4, dans laquelle ledit halotype associé à l'autisme comprend plusieurs SNPs sélectionnés dans le groupe consistant en SNP21 (SED ID:3), SNP22 (SEQ ID NO:4), SNP28 (SEQ ID NO:5), SNP39 (SEQ ID NO:6), SNP46 (SEQ ID NO:7), SNP61 (SEQ ID NO:8), SNP73 (SEQ ID NO:9) and SNP74 (SEQ ID NO:10).

6. Méthode de la revendication 4, dans laquelle ledit SNP associé à l'autisme est le SNP22.

7. Méthode de sélection de composés biologiquement actifs sur l'autisme, ladite méthode comprenant la mise en contact d'un composé test avec un polypeptide ou le gène ATP2B2 ou un fragment de ceux-ci et la détermination de la capacité dudit composé test à lier le polypeptide ou le gène ATP2B2 ou un fragment de ceux-ci.

8. Méthode de sélection de composés biologiquement actifs sur l'autisme, ladite méthode comprenant la mise en contact d'une cellule hôte recombinante exprimant un polypeptide ATP2B2 avec un composé test, et la détermination de la capacité dudit composé test à lier ledit polypeptide ATP2B2 et à moduler l'activité du polypeptide ATP2B2.

9. Méthode de sélection de composés biologiquement actifs sur l'autisme, ladite méthode comprenant la mise en contact d'un composé test avec le gène ATP2B2, et la détermination de la capacité dudit composé test à moduler l'expression dudit gène ATP2B2.

10. Méthode de sélection de composés biologiquement actifs sur l'autisme, ladite méthode comprenant la mise en contact d'un composé test avec une cellule hôte recombinante comprenant un construit rapporteur, ledit construit rapporteur comprenant un gène rapporteur sous le contrôle d'un promoteur du gène ATP2B2, et la sélection des composés tests qui modulent (par exemple qui stimulent ou réduisent) l'expression du gène rapporteur.

11. Méthode selon l'une quelconque de revendications 7-10, dans laquelle ledit gène ou polypeptide ATP2B2 ou un fragment de ceux-ci est un gène ou polypeptide ATP2B2 altéré ou muté ou un fragment de ceux-ci comprenant une altération ou une mutation.

12. Méthode selon l'une quelconque des revendications 7-11, dans laquelle ladite modulation est une activation.

13. Méthode selon l'une quelconque des revendications 7-11, dans laquelle ladite modulation est une inhibition.


### SEQUENCE LISTING

<110> INTEGRAGEN
<120> HUMAN AUTISM SUSCEPTIBILITY GENE ENCODING A TRANSMEMBRANE PROTEIN AND USES THEREOF
<130> B0359WO
<160> 10
<170> PatentIn version 3.1
<210> 1
   <211> 6821
   <212> DNA
   <213> Homo sapiens
<220>
   <221> CDS
   <222> (320)..(4051)
   <223>
<400> 1
   gagccaccac ccctgaccat gtagatgcca gttccaggga gcagcatggg ccccactgaa      60
   tggagactcc tgggtctaca gccctgagcc cctccggccc ctggacctcg tcccacaccg     120
   gaggacacct cttggagctc accaccactg tcaccagccc gcctcggcca cccccacccc     180
   ccgggagccg gagtcggccg cctggtgcca cagctgacca gtgagggtgt gctgaggaca     240
   gccacaagca gccatcaccc ggcagcctct tgtccagcgc tgacccttgg gcccagcccg     300
   agcaaggacc gcagcaaac atg ggt gac atg acc aac agc gac ttt tac tcc      352
   aaa aac caa aga aat gag tcg agc cat ggg ggc gag ttc ggg tgc aca       400
   atg gag gag ctc cgc tcc ctc atg gag ctg cgg ggc act gag gct gtg       448
   gtc aag atc aag gag act tat ggg gac acc gaa gcc atc tgc cgg cgc       496
   ctc aaa acc tca cct gtt gaa ggt ttg ccg ggc acc gct cca gac ctg       544
   gaa aag aga aag caa att ttt ggg caa aac ttt ata cct cca aag aag       592
   cca aaa acc ttc ctg cag ctc gtg tgg gag gcg ctg cag gac gtg acg       640
   ctc atc atc ctg gag att gcc gcc atc atc tcc ctg ggg ctg tcc ttc 688
   tac cac ccg ccc ggc gag ggc aac gaa gga tgt gcg acg gcc cag ggt 736
   ggg gca gag gat gaa gga gag gca gag gca ggt tgg atc gag ggg gcc 784
   gcc att ctc ctc tca gtt atc tgt gtg gtc ctg gtc acg gcc ttc aat 832
   gac tgg agc aaa gag aaa cag ttc cgg ggc ctg cag agc cgc atc gag 880
   cag gaa cag aaa ttt acc gtg gtc cgg gct ggc cag gtg gtc cag atc 928
   cct gtg gct gag atc gtg gtt ggg gac ata gcc cag gtc aaa tat ggt 976
   gac ctc ctc cct gcc gac ggc ctc ttc atc cag ggc aat gac ctc aag 1024
   att gat gaa agc tcc cta act gga gag tct gac cag gtg cgc aag tcc 1072
   gtg gac aag gac ccc atg ctg ctg tca gga acc cac gtg atg gag ggc 1120
   tca gga cgg atg ttg gtg act gct gtg ggt gtg aac tct cag act ggc 1168
   atc atc ttt acc ctc ctg ggg gct ggt ggt gaa gag gaa gag aag aaa 1216
   gac aaa aaa ggt gtg aag aag ggg gat ggc ctt cag cta cca gca gca 1264
   gac ggt gcg gca gct tca aat gct gca gat agt gcg aat gcc agc cta 1312
   gtc aat ggt aaa atg cag gat ggc aat gtg gac gcc agc cag agc aaa 1360
   gcc aaa caa cag gac ggg gca gcc gcc atg gag atg cag ccc ctc aag      1408
   agt gcc gag ggc ggc gac gct gac gac agg aag aag gcc agc atg cac      1456
   aag aag gag aag tcc gtg ctg cag ggc aag ctc acc aag ctg gct gtg      1504
   cag atc ggg aag gcg ggc ttg gtg atg tca gcc atc acg gtg atc atc      1552
   ctg gtg ctc tac ttc act gtg gac acc ttc gtg gtc aac aag aag ccg      1600
   tgg ctg cct gag tgc acg ccc gtc tac gtg cag tac ttt gtc aag ttc      1648
   ttc atc att ggc gtg acg gtg ctg gtg gtc gcc gtg ccc gag ggg ctc      1696
   cct ctg gcc gtc acc atc tcg ttg gcc tat tcg gtg aag aaa atg atg      1744
   aag gac aac aac ctg gta cgc cac ctg gat gcc tgt gag acc atg ggc      1792
   aat gcc aca gcc atc tgc tca gac aag aca ggc acg ctg acc acc aat      1840
   cgc atg aca gtg gta cag gcc tat gtc ggc gac gtc cac tat aaa gag      1888
   atc ccc gac ccc agc tcc atc aac acc aag acc atg gag ctg ctg atc      1936
   aat gcc atc gcc atc aac agc gcc tac acc acc aag att ctg ccc cca      1984
   gag aag gag ggc gcc ctg cct cgg cag gtg ggc aac aag acg gag tgc      2032
   ggc ctg ctg ggc ttc gtg ctg gac ctg aag cag gac tac gag ccc gtg      2080
   cgc agc cag atg cca gag gag aag ttg tac aaa gtg tac acc ttc aac      2128
   tcc gtg cgc aag tcc atg agc act gtc atc aag ctg ccc gac gag agc      2176
   ttc cgc atg tac agc aag ggg gct tct gag atc gtg ctc aag aag tgc      2224
   tgc aaa atc ctc aat ggg gcg gga gag cct cgt gtc ttc cgg ccc cgc      2272
   gac cgg gac gag atg gta aag aag gtg att gag ccc atg gct tgc gat      2320
   ggg ctc cgc act atc tgc gtg gcc tac cgc gac ttc ccc agc agc ccg      2368
   gag ccg gac tgg gac aat gag aat gac atc ctc aac gaa ctc acc tgc      2416
   atc tgc gtg gtg ggc atc gag gac ccg gtg cgg cca gag gtc cca gaa      2464
   gcc atc cgc aag tgc cag cgg gca ggc atc acg gtc cgc atg gtc act      2512
   ggc gac aat atc aac acg gct cgg gcc atc gcc atc aag tgt ggc atc      2560
   atc cat cct ggg gag gac ttt ctg tgc ctc gag ggc aag gag ttc aac      2608
   agg agg atc cgc aac gag aag ggg gag att gag cag gag cga att gac      2656
   aag atc tgg cca aag ctg cgg gtg ctg gct cgc tcc tcc cca acg gac      2704
   aag cat acc ctg gtt aaa ggc atc atc gac agc aca cac act gag cag      2752
   cgg cag gtg gtg gcc gtg acg ggg gac ggg acc aac gac ggg cct gca      2800
   ctc aag aag gcc gac gtg ggc ttc gcc atg ggc atc gca ggc act gac      2848
   gtg gcc aag gag gcc tca gac atc atc ctg aca gac gac aat ttc agc      2896
   agc atc gtc aag gca gtg atg tgg ggc cgc aac gtc tat gac agc atc      2944
   tcc aaa ttc ttg cag ttc cag ctc acc gtc aac gtg gtg gcc gtg att      2992
   gtg gcc ttc aca ggc gcc tgc atc acg cag gac tcc cct ctg aag gcc      3040
   gtg cag atg ctc tgg gtg aac ctc atc atg gac acg ttt gcc tcg ctg      3088
   gca ctg gcc act gag ccg ccc acg gag acc ctg ctg ctg agg aag ccg      3136
   tac ggc cgc aac aag ccg ctc atc tcc agg acc atg atg aag aac atc      3184
   ctg ggc cat gct gtc tac cag ctt gcc ctc atc ttc acc ctg ctc ttt      3232
   gtt ggc gag aag atg ttc cag atc gac agc ggg agg aac gcg ccc ctg      3280
   cat tcg cca ccc tca gaa cat tac acc atc atc ttc aac  acc ttc gtc     3328
   atg atg  cag ctc ttc aac gag  atc aac gcc cgc aag  atc cac ggc       3373
   gag cgc  aat gtc ttt gac ggc  atc ttc cgg aac ccc  atc ttc tgc       3418
   acc atc  gtg ctg ggc acc ttt  gcc atc cag ata gtg  atc gtg cag       3463
   ttt gga  ggg aag cca ttc agc  tgc tct cca ctg cag  ctg gac cag       3508
   tgg atg  tgg tgc ata ttc att  ggg tta gga gag ctc  gtt tgg ggc       3553
   cag gtc  atc gcc acc atc ccg  acc agc aga ctc aag  ttc ctc aag      3598
   gag gca  ggc agg ctc aca cag  aag gag gag atc ccg  gag gag gag      3643
   ctc aac  gag gac gtg gag gag  atc gac cac gcg gag  cgg gag ctg      3688
   cgg cgg  ggc cag atc ctg tgg  ttc cga ggc ctg aat  cgg atc cag      3733
   aca cag  atc cgc gtc gtg aag  gcg ttc cgt agc tct  ctc tat gaa      3778
   ggt tta  gaa aag cct gaa tct  cga acc tcc atc cat  aac ttc atg      3823
   gct cat  cct gaa ttc cgg atc  gaa gat tcc cag ccc  cac atc ccc      3868
   ctc att  gat gac acc gac ctg  gaa gaa gat gcc gcg  ctc aag cag      3913
   aac tcg  agc ccg ccg tca tcc  ctc aac aag aac aac  agc gcc atc      3958
   gac agt  ggg atc aac ctg acg  acc gac aca agc aaa  tca gct acc      4003
   tct tca  agt cca ggg agc ccc  atc cac agc ctg gag  acg tcg ctt      4048
   tag ctgaggaccc tctcgcctgc ccgcccgccc tcatggaccc cgctgccacc          4101
   cgctttccgg gcacccatcc atccaggcac ccaactcacc caagcagcaa cgagcaacaa   4161
   tcggaaacca aatactggag agaaaaccaa cgtttccacc cacagaccct ttctctggct   4221
   gcgatgctgt ttgaactctt tttcacttca aggcaagggg cgggatctcc actgggggct   4281
   tacgggagtg agcggttttc ccaaaacaag cccttcctgg ctcccaccca gacatggacc   4341
   agccatgcac ccgcccagcc accacgtccc ccgcatgaat gtactgtaca ctttcaatcc   4401
   tccccttgtt tggtttttgg gggttgggga ggggtttttg tttgtttgtt tgttttctta   4461
   ggcgggaact gcaaacagac tcttttctga gactatttat ccaatccact ggtctgtgag   4521
   tttttgaaat gcttgcacag catggtctca gttgtataga ttaatttaat aactttttga 4581
   aattgcagag cttaactcgc ctagtagatt tgcaccaatg gaaccgaaga acttcataga 4641
   cactcacaag gttatatcca tttctttgta tctatatcaa cgtatacttt tccgagactg 4701
   tatacgtcca tatagatagg tagatatata tatatatata taaatatata tacatggata 4761
   tataaagttt ctttgccggc atgttgcctt gtttccgctt aaattgctct attttaactt 4821
   atttatgtcc taaaagaaga atgtaatttg tttacaaacc tgtagataac gtctttggct 4881
   atttgtatgg tttaagaaca ctggtggctg agatgctata aaaacagctc ggcccaacag 4941
   acacttcccc tgggttgcat cctggatgtt ttatgatagc catgctctga tttttgcctg 5001
   ctatttccgt tcaataatgt cactaccgtg agaggctcag gcagaagcca aatgctaccg 5061
   agttgccatc ctgaggggtt taacaacatg ctccgtagac gaagggagag gaggagagaa 5121
   ggcttcctgg gtttgcaaca ctaacggcca tccggcccaa ggatgccagg atctgcaaag 5181
   cactgctcga agacttttct ctcaatgaaa ctcgcttgag tttactaaga gcatttcaaa 5241
   aataggttct ctttggcact gtctgtacag agattgaggt agtgttgaaa tattataaat 5301
   ggtattgtgt tgattttttt ttttatttag taacttacag gtttgtttcc ttattaatgg 5361
   cagcatctga gctgttggca tattggatga ggatcagtat ggcttgctgc ttttattttt 5421
   atttttgaag aagaatagcc tttttctctg cactatttag atccgaatga accttatgat 5481
   gtgtatattg agatgtactc agtgtgattt taaaccaaat tgtcttcctg tagtcacaat 5541
   atatactgta gccttttaac agcaagtctt gctttcccaa acagaaagcc attctgaaac 5601
   cctacagtat cacaggtgag aaaaggtggt tattttttcc ccaagacaac agcactagta 5661
   atcccactta ataagagctt atttaattgg atgtcagcct cttaactgct aagcactttg 5721
   tgggtctcag cgtttttcat aaaagaactt ttgtatttaa tacaaagttt gctttgagac 5781
   ttttcagcat atgatctttt ttccataaac ttgtacagtg caaaagacat tttgaatacc 5841
   atgatcgatg atgtcccatg cttcgaggaa aaccaaacac tttccgcctc tcttgcaaaa 5901
   tccattcctc atgctgaccc tcctcacgat ggctgtgtca gcccagcccc ttcccttctc 5961
   caggcccaga gaactcttcc acaaacaaga tgagagccac tcgggaaaag agccatagtc 6021
   aactgggagg gcctacatct ggatggcggt ggaaaaactt gagggtttgg ggttcaaagt 6081
   cagcccatcc cacctggcaa aatcctcctg gaaggaggac cttcaagagc gcatcacctg 6141
   aatgtcgtga agaagtatct ctgaatgtat ccaggagagg aactgcataa ccaaaggggt 6201
   gaccagccct cagatgtgct tattggattc cagtacaaac gccaccaaag ccagcccact 6261
   gctctcctac aaggaaggaa agatctgcac gtgtaaaaca tggggcagcc ttggaacatg 6321
   gtgttttttg gagtttcctt tctcacagtt ttccatctcc ccacttcttt gatcagtcat   6381
   gtgtccgtga cctcgttcca tgacatcagg atagctgtgt ttgcacacca tgctccatgt   6441
   tcattcggag ccaggagggg ttctcagtgg agcctggctt agggaacagg gagcgatgga   6501
   agaatgccaa cattagcgtt ggtcttctct tgtcaggaat gaaggatgct tgcacacatg   6561
   caccccctca ctctcacact tgcacacata cacacacaca cacacgaaat ggttggtttg   6621
   tcaaaactca ctgtagtaca taaagcttgc actctgcgtc ctatatctag cagcatgggg   6681
   tacgtttggc agttcactcc attagggggt aaataattta tgaccattca tctgttttta   6741
   tgaatttttt tatctagaca ataatgtaaa taaagaactc accatctctg ttcatttaat   6801
   actaaaaaaa aaaaaaaaaa                                                6821
<210> 2
   <211> 1243
   <212> PRT
   <213> Homo sapiens
<400> 2
<210> 3
   <211> 1001
   <212> DNA
   <213> artificial sequence
<220>
   <223> SNP21
<220>
   <221> misc_feature
   <222> (501)..(501)
   <223> C/T
<400> 3
   ggcggacccc ctgaccacca cgccctgggg gaccctgggc gacctggcct gcagcttctg     60
   tccagcctca cacggcccct gtgggtgggt gcccccagcc ctgagcgaca gtcccctcac    120
   tcacctctgc cctcagagcc acccttcccc accctctcag ggaaatcccc ctggagacca    180
   cggtgggtgt gacccctacc acttccccgt gctgccctga gagggcacaa tgggacctct    240
   ctgaggaggc cgtgctgttc cttagcagcc acaggagtgg gtgtgcaagt ggctggtgag    300
   agctggccgg gacccctctg tagccaccct ccctcagcac cagagctggg ccctgggggg    360
   cagcacaagc acactcacct ccatcccctg gcccgccccg gccaggcctg ggcccagccc    420
   ccaagagcct cctgtacctg tgtctggatc cgattcaggc ctcggaacca caggatctgg    480
   ccccgccgca gctcccgctc ygcgtggtcg atctcctcca cgtcctcgtt gagctcctcc    540
   tccgggatct cctccttctg tgtgagcctg cctgcctcct tgaggaactt gagtctgctg    600
   gtcgggatgg tggcgatgac ctgcaaggga ccctgtctgt caggacggtg gggctgtcct    660
   tcccagtggc cttctctccc tgacacccgc tcctctacct ccctcctcct acccgctcac    720
   ccaggcatgt gatcaatcta ctccttcacc cacctcacct gttcctcagc ccagtctttg    780
   acaatgcctt tttttttttt tttttttttt ttgagatgga gtttcactct tgttgcccag    840
   gctggagtgc aatttgcttt ttaattcatt cattcagagt cagggtacat agtggacaat    900
   cttggcctct aggagttgga cagatgtggc tttgaatacc ccagacaacc ctggtcctgt    960
   ggcctcgggc aagtcattta acctcaccga gctttggttt t                       1001
<210> 4
   <211> 537
   <212> DNA
   <213> artificial sequence
<220>
   <223> SNP22
<220>
   <221> misc_feature
   <222> (464)..(464)
   <223> C/T
<400> 4
   gctcctgcac cctccacagc agctgagcag ccacccgcat catcctggag cagcaggcta 60
   agggaggcag aactgacaac ggggaaagta ggtcagagga cccatgcaga cagtgataga 120
   gaaccctggc ggtgatgcca aacatgacga cgtttttgtc ttcttccccc accccaccca 180
   atttgggaat caagggagat cagcgatgct ttattctgct ccccaggact tcctaaaata 240
   actgggatgc tgataatgat ctcctcctgc agacctggca gagaggggaa cagggcgatt 300
   cccagaacgg gcttcagcca tgtgggcaca gccctgtggg gtgggcttgg attgagatgt 360
   gtgtaatgca attatagtca aaaagaattc ttgggatctg ccaggaaggc tgaggaaacc 420
   tctccacagt gatgagtctc aattaagcag tgctttttag cacyggaggc aaagctgaaa 480
   tgtcatttcc agatgttggt ggattgagtc ttttcaggtg gcataaagga caggggt 537
<210> 5
   <211> 762
   <212> DNA
   <213> artificial sequence
<220>
   <223> SNP28
<220>
   <221> misc_feature
   <222> (562)..(562)
   <223> C/T
<400> 5
   aggatgaaga ataaaatcca tacagctcct gcctggcacc cacgcacctc agcctgcacc 60
   atgcccttct tgcttatgga gctctggcca caggcccttt gcacaggctg agctctctgt 120
   ctctgtagtc ttccctcagc ctagctaact cctcttcatc cttcagacct ccattcagtg 180
   tcacttcttg gggaagcctt tcctgagcat gctggctggg ctgtccctgc tgtcccttac 240
   taacctccca cagctctgtg cacggcatgc acctatctct cagcaagcac aacttgacac 300
   gtaactgtgg ggttcctgga ataggggctg cctcctggct gggctgggag agacccttat 360
   ctggttttgc ccgttgtacc cccagcacat tcacagtgcc tggcacctac taggactcta 420
   taactactgc ctgaatcaat gaacaaaagc aaagatgtgt gggaatttac agctgaaact 480
   gcatttgagg ttgcttggag ggcatgccaa atctcaattt cttcctaaaa tttaggctca 540
   gctcccctcc tggcatctaa gytcctgaag gagtcacagg catggtgtca cctctcattc 600
   tgggagtctt gcccgcctcc agcaggggat aagctggtat cagagctggc agcagaggga 660
   gatcaatcac agaaggaaaa agagaccagg gctgctccac tagaccagag aagggctctg 720
   cttctgtcct catcagaaaa acctagagag ggattcttta gg 762
<210> 6
   <211> 401
   <212> DNA
   <213> artificial sequence
<220>
   <223> SNP39
<220>
   <221> misc_feature
   <222> (201)..(201)
   <223> C/T
<400> 6
   ggaattttaa ctcaaatacc aaaatctagg gcgatttgcc tatcacgcat ccatttattc      60
   cacttcctcc agcaagttca cagcactatg taaaatgctt agaaccagtg gaggtttttg     120
   tgagttctgg aaaacaagag gagggaaaaa tcaatggaga aaagcaggag tgaagcagat     180
   agccactgag aagaagaaga yggcaggctg acgggaaagt acagcggccg agtgtctgct     240
   ggcgagctgt gcgtcagggc ccacttgcac aaatagtaag gcagaactag tggcccccta     300
   gacccagggg gcgtttagag gtcctgggaa acataaactc tccatcctaa gacaacacta     360
   caccctccca ctcctaatca aggcccttac attccaccat t                         401
<210> 7
   <211> 1001
   <212> DNA
   <213> artificial sequence
<220>
   <223> SNP46
<220>
   <221> misc_feature
   <222> (501)..(501)
   <223> A/G
<400> 7
   aacccctgat tttagtcagg gagatggatt tgagactgat ctcccatctt gcctgctgca    60
   gcgcctgatt aaagccttct tccctggcaa cagtcattgt ctcagtgatt ggctttctgt   120
   gtggcaagca gcaggaccca gactgaatcc ctggcatttt ggtaacacct tcttgcattt   180
   gaggatggga tattggtgtc tctttggttt tctctttcac ctaactacta ctccttacat   240
   ttaagaatgc ttcttataac acagtttcca gtcatggttc tcaatacccc tcagtagttt   300
   taagtgtagt aatctgcagt gaagtcagtc ctctaggcat gggttctctc cattcaactt   360
   ctagaagttt aacttggtac cgaatctctt tgtgatgccc cagattgtgc ttgcaagcat   420
   caaaaacctc ttcttcacag gactacttag tcatgtttcc cctaggctgt atcacggaaa   480
   cttttgtttt ttgtatctaa rtatttacct accttgtgcc tgctgtgttt tacctggtta   540
   ggcttagact cttatctgtc ctgatctctc tgatcctgat tttactactc atcctggttt   600
   agcatcaccc attgagggag gcagaaggca gagaaactct aggcagacag gggcaggtcc   660
   ccagtggaaa ccccaccttc aagccagaag tagcctgaaa cccttggccc agggtgagaa   720
   cttctattct cctgtttgcc tgctctctcc tgagtcattc tttctgaata atgtcttttt   780
   accaattgaa tgttgccttt tgcaaaacta cctatggcca gccctgtccc cccatcctgt   840
   gtctataaag actccaggct cagctggcag agaggagaag cagctggatg ttggggagag   900
   gcgacttgcc ttcagagatg gtggctggat ggcaaagaga ggggcaactt gactttggag   960
   aagaggggca gagaggcaaa ttgacttcag gggagagtga c                        1001
<210> 8
   <211> 837
   <212> DNA
   <213> artificial sequence
<220>
   <223> SNP61
<220>
   <221> misc_feature
   <222> (337)..(337)
   <223> C/T
<400> 8
   cattcaggtt ccccaccagt caggtccaga tgcaggacgg catgggggag gctttaggcc 60
   tctgtagatg ggcactgtat gagtgacccc atctccaggg tccagtaaca atgccagtgt 120
   tgcatccaga gtcatagagg gacagcatgg cctggatgcc cacgtacatg gacagggtgt 180
   tgaaggtctc aaacatgatc tgggtcatct tctctctgtt gcccttgggt ttcacaaggg 240
   cctccgtcag cagcaccagg tgctcctccg gggccacacg caactcactg tagaaggtgt 300
   ggtgccaaat cttcttcatg tcatcccagt tgatgaygat gccacgctca atggggtcag 360
   agtcccaatc ttcagcgtca ggatgccatg cttgctctgg gcctcatcac ccacgtagga 420
   gtccttctga cccatgccca ccatcgtgcc ctggtgccag gggcgcctaa ggatggaggg 480
   gaacacggct acagggggcg tcgtccccag caaatccagc tttgtacatg ccggagacac 540
   tgtcaatgac cagcatggca atctcttctt ccattgtgac aggcggagga gcagggcggc 600
   agagcagtag gaggacatgg tgcacgggct ggcggcagcg actgtgtgat actcaacgtc 660
   attaaccatc aggaaaatgc atatcaaaac tggagtacca gacaggcata gtggcttatg 720
   cctatcatct cagcactttg ggaggccaag gcgggaggat cacttgagtt tgaggaattc 780
   gagaccaacc tgggcaacac tgcaagacct catatctact aaaaattagc caggcgt 837
<210> 9
   <211> 401
   <212> DNA
   <213> artificial sequence
<220>
   <223> SNP73
<220>
   <221> misc_feature
   <222> (201)..(201)
   <223> C/T
<400> 9
   tctatgtaac catgagttta gagaaaatga caaaaaagaa ggtggtaaag ggtgagaaac 60
   tagaaggtgt gaccactgcc agccaccctg ctcccatagg aggagccaca ttttcaccag 120
   ggcccctgca cctgctcctc agtcctctgt ttcctccttg aagtgatcct cacctggcgc 180
   tcacttctct ctggcaggga yagcagttgc agggtggtgg gatcatttgt ggaatgccgg 240
   gactgggttc ttaccttgga aatgctgacg aatgggaccc taccacgcca cattgccaca 300
   gcaacaaagc ccaggcctga gctttgcagg gtcagcactt gtgggctcct gacctccttc 360
   cccagcctcc tcgaggccct cccgcagtac tggctacctc c 401
<210> 10
   <211> 401
   <212> DNA
   <213> artificial sequence
<220>
   <223> SNP74
<220>
   <221> misc_feature
   <222> (201)..(201)
   <223> A/C
<400> 10
   ttcaaggaac cttatttaga aattaatcta gcatgttgct gcttagaaca atggcaattc     60
   taggaaggtt ccaacttccc gattcttctt tttcatggtt atctttgtaa ttgatgtatc    120
   atttcctcta actcctcttt agtctgtgtt tttctgtgtc ctcatataat tcctcagctt    180
   cttccttgag aagttggata mtgatatcat cacacgttgg ataagccttt tcttaatggt    240
   catgacactc ttaaagccac tcaccttccc ctccctcagt tttggccacc ttcttgactg    300
   tttcaggctt gatggcatcc cttgccaatg ggatgcaagt actgagtgca ggagccacct    360
   aagtaattag tcttattttt tcccttgtga ggattcggag t                        401